# EUROPÄISCHE PATENTSCHRIFT

(11) **EP 2 630 920 B1**
(45) Veröffentlichungstag und Bekanntmachung des Hinweises auf die Patenterteilung: **25.11.2015**
(21) Anmeldenummer: 13002300.5
(22) Anmeldetag: 03.07.2007
(51) Int. Cl.: A61B 17/04, A61B 17/064, A61B 17/12, A61B 17/74, A61B 17/86, A61B 17/88, A61C 8/00, A61F 2/34, A61F 2/36, A61L 24/00, A61L 27/50

(54) **Medizinisches Implantat**
Medical implant
Implant médical

(43) Veröffentlichungstag der Anmeldung: 28.08.2013
(62) Teilanmeldung aus: 07720221.6
(73) Patentinhaber: Synergy Biosurgical AG, 6301 Zug (CH)
(72) Erfinder: Bähre, Wolf-Friedrich, 8700 Küsnacht (CH); Ruffieux, Kurt, 8800 Thalwil (CH)
(74) Vertreter: Lusuardi, Werther

(56) Entgegenhaltungen:
- EP-A- 0 358 601
- WO-A-02/069817
- US-A- 4 525 147
- US-A1- 2005 107 872

## Beschreibung

Die Erfindung bezieht sich auf ein medizinisches Implantat gemäss dem Oberbegriff des Patentanspruchs 1.

Biokompatible, thermoplastische Materialien für osteosynthetische und ähnliche Verfahren für Befestigungszwecke an menschlichen oder tierischen Knochen zu verwenden ist eine bekannte Technik und wurde auf verschiedene Weisen versucht, z.B. mittels äusserer Wärmeapplikation analog zu einer Heissleimpistole (z.B. US Patent 5290281) oder durch Verflüssigen des Polymers mittels Ultraschallenergie gemäss der WO2006/002569 WOODWELDING. Diese Techniken sind allerdings mit Nachteilen behaftet: Die Erwärmung mittels äusserer Wärmequellen - analog zu einer Heissleimpistole - führt dazu, dass ein Implantat sehr schnell eingebracht werden muss um nicht wieder abzukühlen währenddem es die Verbindung mit dem Knochen eingeht, weil es typischerweise eine nur geringe Wärmekapazität aufweist, und nur im erweichten Zustand kann das thermoplastische Material z.B. in die Zwischenräume im Knochen eindringen. Sobald das Material wieder abgekühlt ist erfolgt keine weitere Verbindung mit dem Knochen. Auch das notwendige übermässige Erhitzen des thermoplastischen Materials - um eine vorzeitige Erstarrung zu verhindern - ist nachteilig, weil sowohl das Material als auch das (Knochen-) Gewebe dadurch geschädigt werden. Ferner wird nicht - wie es idealerweise wünschbar wäre - nur die Uebergangszone zum Gewebe im Implantat erhitzt sondern auch Zonen, welche nicht erhitzt und erweicht werden sollen, weil sie zwischen Wärmequelle und Zielbereich im Kunststoffmaterial liegen. Das nachträgliche Entfernen des abgekühlten Thermoplasten ist schwierig und ohne übermässige Erwärmung des umliegenden Gewebes kaum zu realisieren. Diese Nachteile bestehen auch bei der Bestrahlung mit elektromagnetischer Strahlung, z.B. Infrarotlicht.

Die vorstehend im Zusammenhang mit einer äusseren Wärmequelle erwähnten Probleme bestehen zwar bei der Verflüssigung mit direkt aufgebrachter Ultraschallenergie nicht, der gravierende Nachteil hier besteht aber darin, dass der Knochen genügend mechanischen Widerstand leisten muss, damit der (vibrierende) Thermoplast an der Kontaktzone mit dem Knochen erweicht wird und es besteht das Risiko einer mechanischen Schädigung des Knochens. Grundsätzlich muss ein möglichst dichtes Knochengerüst vorhanden sein, um ein sicheres Aufschmelzen des Thermoplasten zu gewährleisten. Gerade dort, wo ein gutes Einschmelzen des Polymers in den Knochen wünschenswert wäre, vor allem im Bereich von osteoporotischem Knochen, kann so nur in unzuverlässiger Weise ein Aufschmelzen des Polymers erreicht werden und es findet bei fehlender Schmelze keine mechanische Verbindung statt. Ein weiterer Nachteil der Ultraschalltechnik besteht darin, dass nach Aushärten des Polymers nach erfolgter Verbindung mit dem Knochen keine Verflüssigung mehr erreichbar ist, d.h. das Implantat kann nicht ohne grösseren Aufwand (aufbohren, herausreissen, schmelzen, warten bis degradiert) wieder entfernt werden.

Hier will die Erfindung Abhilfe schaffen. Der Erfindung liegt die Aufgabe zugrunde, ein medizinisches Implantat zu schaffen, welches durch innere Wärmentwicklung zum Erweichen gebracht werden kann. Das Risiko einer Schädigung des Gewebes durch übermässige Wärme, (im folgenden zumeist am Beispiel von Knochen erläutert), wird insbesondere im Vergleich zum Stand der Technik, wo eine äussere Wärmequelle benutzt wird, reduziert. Die Abhängigkeit des Fixationsergebnisses von der Knochenqualität wird deutlich reduziert. Das elektrisch leitende Polymer kann wieder mittels elektrischen Stroms aufgeweicht und zumindest teilweise entfernt werden.

Der nächstliegende Stand der Technik wird in EP 0 358 601 offenbart, das ein Implantat beschreibt mit einem thermoplastischen Werkstoff, der mittels eingelegter Kohle- oder Kupferfäden leitfähig gemacht wurde.

Die Risiken bei der Verwendung des erfindungsgemässen medizinischen Implantates sind deshalb reduziert, weil die eigentliche Wärmentwicklung (zur Erweichung des Polymers) im Inneren des Implantates und am Übergang zwischen Implantat und Knochen erzeugt wird. Die Energieabgabe durch den Strom wird dort, wo das Implantat den Knochen berührt im Inneren des Implantats konzentriert und wegen der verhältnismässig geringeren funktionellen Querschnittsfläche des leitenden Implantats erfolgt dort (idealerweise an der Berührungsstelle) bei grösster Stromdichte die grösste Wärmeentwicklung. Dieser Effekt kann durch ein geeignetes Design des Implantats und der Elektrode noch verstärkt werden. Ferner hat es sich erwiesen, dass nach schmelzen des Polymers dieses in geeigneter Zusammensetzung seinen elektrischen Widerstand um bis zu Faktor 10³ - 10⁸ reduzieren kann und dadurch durch den fliessenden Strom nicht mehr weiter relevant erhitzt wird. So ist das System thermisch und mechanisch zu einem gewissen Teil selbstregulierend und schont das umgebende Gewebe. In anderen Ausführungen hat sich eine entsprechende Erhöhung des Widerstand als unter Umständen ebenfalls vorteilhaft erwiesen (zum verschmelzen von Polymerteilen gegeneinander z.B.).

Der Knochen kann ferner dadurch geschützt werden, dass die elektrische Leitfähigkeit des Implantats oder des Übergangs Implantat-Knochen geringer gewählt wird als diejenige des Knochens, wodurch sich dieser auch weniger erwärmt.

Bei Bedarf ist es auch möglich den Knochen selektiv mit Kühlelementen, Spülflüssigkeit oder Gas/Luft zu kühlen.

Das erfindungsgemässe medizinisches Implantat kann in verschiedenartigen Implatatformen realisiert werden, insbesondere als Schraube, Stift, Clip, Stecker, Platte, Nagel, Spickdraht, Cage, Pedikelschraube (-nagel), Stent, Expander, Piercing, Hautverbindung, Medikamententräger, Genmaterialträger, Träger von bioaktiven Faktoren, (z.B. Wachstumsfaktoren, Knochenbildungsfördernde Substanzen Schmerzmitteln, usw.) Träger von anderen Implantaten, Dübel, Klammer, Perle, Zahnimplantat, Zahnwurzelimplantat, Schlauch, Rohr, Faden, Faden in Schlauch oder Rohr, Gewebe, Geflecht, Gestrick, Strumpf, Band, lose Fasern, Faserknäuel, Faserflocken, Granulat, Kette, Anker mit oder ohne Fadengleitöse. Das Implantat kann nach Implantation auch als Elektrode im oder auf dem Körper dienen für Zwecke der Diagnostik, Stimulation oder Monitoring. Die gleichen Elemente können auch mit einem eingearbeiteten Draht, Stift oder einem anderen Stromleiter (aus gleichem oder anderem Material, z.B. Metall), welche als temporäre Stromzuführung aber nicht direkt zur Erwärmung dienen und die später fakultativ entfernt werden können. Diese stromleitenden Elemente können nachher auch belassen werden und bioresorbierbar ausgelegt sein (z.B. aus Magnesium).

Die Erfindung löst die gestellte Aufgabe mit einem medizinischen Implantat, welches die Merkmale gemäss dem Anspruch 1 aufweist.

Bei dem erfindungsgemässen Medizinischen Implantat wird der Effekt benutzt, dass in elektrischen Leitern oder am Übergang zwischen elektrischen Leitern beim Fliessen eines elektrischen Stroms am Ort des grössten (z.B. Ohmschen) Widerstands (in einem Stromkreis) Wärme entsteht. Spezifisch für das erfindungsgemässe medizinische Implantat ist, dass elektrischer Strom für die Erwärmung eines Polymers, vorzugsweise eines thermoplastischen Materials, verwendet wird, welches selbst elektrisch leitend ist oder durch Zusätze elektrisch leitend gemacht wird. Mit Hilfe eines solchen thermoplastischen, elektrisch leitenden Polymers gelingt es überraschenderweise durch Aufweichen des Thermoplasten und geeignetes Design des Implantats mechanische Verbindungen von, zu und mit menschlichem oder tierischen Knochen zu erreichen, vorzugsweise während einer chirurgischen Operation. Da die grösste Wärmeentwicklung dort entsteht, wo sich der grösste Widerstand in einem Stromkreis und somit der grösste Spannungsabfall findet, ist es auch möglich den Erwärmungsprozess spezifisch derart zu steuern, dass sich das Material nur an gewissen bevorzugten Stellen erweicht. Es ist zu beachten, dass der Strom sich den Weg des geringsten Widerstands sucht und einen Stromkreis bildet. Der Ort des grössten Widerstands entspricht dem Ort grössten Widerstands in einem solchen Stromkreis und das Implantat muss so ausgelegt werden, dass der Strom gezwungen wird, durch die gewünschten Bereiche im Implantat zu fliessen und diese nicht anderwärtig abfliessen kann. Ansonsten wirkt das Implantatmaterial als partieller Isolator und wird wenig von Strom durchflossen und entsprechend wenig erweicht. Der menschliche oder tierische Körper, und hier insbesondere der Knochen, hat sich als sehr geeigneter elektrischer Leiter erwiesen, um als Weiterführung des elektrischen Stromkreises zu wirken und erwärmt sich in der hier beschriebenen spezifischen Anwendung überraschend wenig. Dies wird durch Polymere zusätzlich begünstigt, welche ihren Widerstand in geschmolzenem Zustand reduzieren.

So kann bei einer besonderen Anwendung das Implantat an einem Pol (bei Gleichstrom) oder am Phasenleiter (bei Wechselstrom, radiofrequentem Strom) des Stromkreises angebracht werden, währenddem am Körper (Knochen) über eine grossflächige Elektrode der andere Pol oder der Nullleiter angebracht wird. Der Strom fliesst über eine Elektrode zum Implantat, dann durch das Implantat und an der Berührungsfläche/Stelle mit dem Körper, z.B. dem Knochen in denselben weiter und danach wieder über eine neutrale Elektrode hinaus. Der Strom wird das Implantat am gewünschten Ort grössten elektrischen (ohmschen, induktiven oder kapazitiven oder anderen) Widerstands resp. Spannungsabfalls im Stromkreis erwärmen und aufweichen.

Alternativ kann der Strom auch ohne durch den Patientenkörper zu gelangen direkt zwischen zwei geeignet angebrachten Polen ("bipolar") durch das Implantat geleitet werden, welches dadurch wiederum an der Stelle/Region des grössten Widerstandes erwärmt und aufgeweicht/geschmolzen wird.

### Für die nachfolgenden, in der gesamten Beschreibung häufig verwendeten Begriffe gelten die folgenden Definitionen:

**Strom:** Die Erfindung benutzt zum erwärmen und aufweichen des Implantats elektrischen Strom welcher durch das Implantat fliesst, geeigneterweise mittels Elektroden appliziert. Der Strom kann ein ohmscher Strom sein, bei welchem Elektronen fliessen wie bei Gleichstrom in Metall. Als zu verschiebende Ladungsträger kommen auch Protonen oder andere geladene Teilchen in Frage. Der Strom kann aber auch eine Ionenverschiebung sein, wie dies bei Stromfluss in einer Salzlösung der Fall ist. Auch chemische Reaktionen, welche ähnlich einer Batterie das Verschieben von Elektronen oder Ladungen erlauben können sind möglich. Insbesondere sind auch der induktive oder kapazitive Strom eingeschlossen, kapazitive Ladungsverschiebungen sind in dieser Anwendung die bevorzugte Art des Stromflusses. Der Strom kann auch auf unterschiedliche Weise fliessen, z.B. im Knochen als Ionenstrom und Protonenstrom, im Polymer z.B. gleichzeitig als Elektronenstrom. Der Strom oder die elektrische Spannung kann als Gleichstrom, Wechselstrom oder, bevorzugt, als hochfrequenter Wechselstrom (Radiofrequenz) verwendet werden. Auch ein Funkensprung kann für die erfindungsgemässe Anwendung genutzt werden.

**Schmelzen / Aufweichen / Erweichen:** Unter Schmelzen, Erweichen oder Aufweichen des Implantatmaterials im erfindungsgemässen Sinne wird die Erweichung des Implantats durch den Stromfluss selbst oder durch die dadurch erzeugte Wärme verstanden, bis sich das vorher nicht in nützlicher Weise (typischerweise von Hand) plastisch verformbare Implantat im Körper mit moderater Kraftanwendung (typischerweise von Hand) verformen lässt und in erfindungsgemässer Weise verwenden lässt.

**Widerstand und Leitfähigkeit:** Mit "elektrischer Widerstand" oder "Leitfähigkeit" ist der Oberflächenwiderstand (Ohm/square), die Volumenleitfähigkeit (S/cm) oder der absolute Widerstand, für die jeweils verwendete Stromart, gemeint. Diese Definitionen sind austauschbar und nicht einschränkend gemeint. Insbesondere strebt die Erfindung eine ausreichende Leitfähigkeit des Materials an, um genügend Stromfluss für die notwendige Aufweichung zu erreichen, andererseits ist ein gewisser Widerstand notwendig um einen genügend grossen Spannungsabfall zur erreichen und damit eine genügend grosse Energiefreisetzung im Implantat um dieses zu erweichen. Idealerweise scheint eine deutlich geringere Leitfähigkeit als im umgebenden Gewebe erstrebenswert, da dieses dadurch geschont wird (wenig Erwärmung dort). Letztlich ist die Leitfähigkeit eine Funktion von Stromart, Spannung, Materialquerschitt und Volumenleitfähigkeit / Widerstand des Materials selbst und muss für die jeweilige Anwendung angepasst sein. In der komplexen Wechselstromrechnung wird der Begriff "Widerstand" durch eine komplexe Grösse, der sogenannten "Impedanz" mit den Komponenten "Resistanz R" und "Reaktanz X" ersetzt.

**Neutrale Elektrode:** Unter der "neutralen Elektrode" ist im Falle von Wechselstrom der Pol gemeint, welcher mit dem Nullleiter oder der Erdung verbunden ist.

**Monopolar:** Unter "monopolar" ist eine Anwendung zu verstehen, in welcher durch den Körper der Stromabfluss (via Nulleiter, "neutrale Elektrode" oder Erdung auf der Haut oder sonstwo am Körper mittels grossflächiger Elektrode) erfolgt und die Wechselspannung typischerweise via Implantat eingespeist wird. Die Pole lassen sich auch umkehren.

**Bipolar:** Unter "bipolar" ist hier die direkte Ein- und Ausleitung des Stroms durch zwei Elektroden zu verstehen, welche in der nahen Nachbarschaft des Implantats (z.B. Elektrische Pinzette mit zwei Polen) angebracht werden. Der Vorteil ist hier dass Stromfluss durch den Körper reduziert oder vermieden werden kann.

**Organische Halbleiter:** "Organische Halbleiter" repräsentieren eine Gruppe von leitenden Polymeren, einerseits die Gruppe der Ladungs-Transfer-Komplexe ("charge transfer complexes") und andererseits die Polyacetylene, Polypyrrole, Polyaniline usw., sowie deren Derivate. Diese Polymere können stets in beliebigen Mischungen oder in reiner Form ihrer Trans- und Cis- Formen vorhanden sein.

**Selbst leitendes Polymer:** Darunter sind Polymere zu verstehen, welche ohne weitere Zusätze elektrisch leitfähig sind, zusätzlich eingeschlossen sind hier auch Co-Polymere (z.B. Copolymer zwischen Lactid mit Pyrrol), welche ebenfalls elektrisch leitfähig sind.

**Leitfähig gemachtes Polymer:** Darunter sind Polymere zu verstehen, welche mit Zusätzen, typischerweise Pulver im Mikro- oder Nanometerbereich, selbst leitende Polymeren, niedermolekulare Substanzen oder Flüssigkeiten versehen sind und mittels dieser Zusätze elektrisch leitfähig gemacht werden. Auszuschliessen aus dieser Gruppe sind ausdrücklich Polymere welche mittels weiterer makroskopischer Bauteile, namentlich Fasermatten, Endlosfasern, Drähten, Fäden, Nadeln usw. versehen sind und das Polymer so selbst nicht mehr leitfähig ist sondern nur das im Polymer enthaltene zusätzliche Bauteil, durch dessen Erwärmung das umgebende Polymer aufgeweicht wird.

Das erfindungsgemässe medizinische Implantat gestattet die Lösung verschiedenartiger Aufgaben, wovon einige im Folgenden näher beschrieben werden.

### Aufgabe A: Selektives oder globales Erwärmen und Aufweichen oder Verflüssigen von medizinischen Implantaten mittels elektrischen Stroms während der Implantation derselben.

Dabei wird am Beispiel eines geraden Pins ein Stromkreis hergestellt via eine Elektrode, welche am einen Ende (vom Knochen weggewandte Seite) eines elektrisch leitenden Pins angeschlossen wird, über den leitenden Pin selbst, dann über die Kontaktstelle zum Körper (z.B. Knochen) und über den Körper zu einer neutrale Elektrode. An der Kontaktstelle zwischen Implantat und Knochen findet der grösste Spannungsabfall (grösster Widerstand) statt und der Thermoplast erwärmt sich und wird weich bis flüssig. Der Kern des Pins ist so konzipiert, dass er sich nicht oder nur teilweise erwärmt und hart bleibt. So kann der Pin nun in ein vorgebohrtes Loch gestossen werden, welches z.B. ein Untermass aufweisen kann und der erwärmte, weiche leitfähige Thermoplast wird in die Zwischenräume im Knochen gepresst. Nach Ausschalten des Stroms kühlt der Thermoplast aus und wird schnell (< 1-2 Minuten) hart, die mechanische Verbindung ist geschaffen.

### Aufgabe B: Selektive oder globale Erwärmung eines einen Thermoplasten umfassenden Implantates um eine Verformung während der Implantation desselben zu erreichen.

Dabei wird Beispielsweise ein leitender Pin in seinem Verlauf mit einer Zone höheren Widerstands versehen und wiederum in den Stromkreis eingebracht, an der Zone mit grösserem Widerstand wird sich der Pin erwärmen. An dieser Stelle kann sich der Pin verformen.

### Aufgabe C: Erreichung einer lokalen Fixation eines einen leitfähigen Thermoplasten umfassenden Implantates im Körper.

Ein Pin wird durch ein geeignetes Herstellungsverfahren, z.B. mittels Spritzgiessen, mit einer Eigenspannung versehen. Durch das Erwärmen des gesamten Pins relaxiert der Thermoplast und der Pin wird kürzer und nimmt im Durchmesser zu, was zu einer Fixation im oder am umgebenden Gewebe führt.

### Aufgabe D: Erreichung einer lokalen Verbindung zwischen mehreren, einen leitfähigen Thermoplasten umfassenden Implantaten durch Verschweissung derselben untereinander.

Diese besteht im Verbinden von zwei thermoplastischen Implantatteilen, welche separat in den Körper eingebracht werden könne. Dabei muss gewährleistet sein, dass der benötigte Strom durch beide (oder eine Vielzahl) zu verbindende Implantatteile fliessen kann. Nach dem Einbringen der beiden (oder der Vielzahl) Implantatteile wird der Strom eingeleitet, die Implantatteile erweichen an ihrer Kontaktstelle und können durch ein Aufbringen von Druck zusammengefügt werden. So kann z.B. auch ein Faden verklebt werden um auf einen Knoten zu verzichten, dabei wird z.B. mit einer bipolaren Stromquelle der Strom direkt durch die Verbindungsstelle geführt.

### Aufgabe E: Auskleiden von Hohlorganen.

Blutgefässe, Darm, Magen, Knochen, Harnleiter, Blase, Uterus, Gallenblase, Tuben, Vagina, Urether usw. können mit einem aus dem beschriebenen Implantatmaterial hergestellten medizinischen Implantat, beispielsweise in Form eines Stents ausgekleidet und damit z.B. mechanisch augmentiert werden. Dabei kann ein Stent, welcher unter Eigenspannungen steht durch eine Erwärmung expandieren, oder der Stent kann im erweichten Zustand unter zu Hilfenahme von mechanischem Druck, z.B. durch Aufdehnen des Ballons, verformt werden.

### Aufgabe F: Klemmen oder Ummantelung von Weichteilen oder Knochen.

Es kann z.B. ein Magenband aus dem Implantatmaterial geformt werden (als offener Ring) und durch Stromzufuhr verformbar gemacht und zu einem geschlossenen Ring verbunden werden. Analog lässt sich ein Polymerband auch als Cerclagematerial verwenden.

### Aufgabe G: Hautverschluss.

Besonders Polymere mit vorzugsweise niedrigem Schmelzpunkt können auch direkt auf die Haut geklebt werden, z.B. zum mechanischen Hautverschluss oder z.B. als EKG Elektrode.

### Aufgabe H: Herstellung von Implantaten, welche nach ihrer Einführung in den Körper durch Durchschneiden des Implantatmaterials veränderbar sind.

Das hier verwendete und beschriebene Implantatmaterial kann auch dazu verwendet werden, Implantate herzustellen, welche selektiv mit elektrischem Strom durchtrennt oder eröffnet werden können, so kann z.B. ein elektrisch leitender Faden auch mittels Elektrokauter insbesondere mit grosser Spannung oder Strom und einer vorzugsweise kleinen "scharfen" Elektrode durchschnitten werden. Eingeschlagene oder eingeschmolzene Pins können so z.B. an der Knochenoberfläche abgeschnitten oder anmodelliert werden bis sie plan mit der Oberfläche der Knochens sind. Medikamententräger können so eröffnet und Wirkstoffe freigesetzt werden.
Für das Aufweichen bzw. Verflüssigung des erfindungsgemässen medizinischen Implantates eignet sich jede Art von Strom, insbesondere Gleichstrom, Wechselstrom, Induktiver Strom (Mikrowelle), Drehstrom, Mehrphasiger / mehrpoliger Strom, typische Strommuster des Elektrokauters.

Die Stromleitung kann verschienartigen realisiert werden, wie z.B. Stromleitung direkt mittels 2 Elektroden ("bipolar") durch den Thermoplast oder ("monopolar") via Körper, Stromleitung via kapazitiven, ohmschen oder ionischen Strom, Stromleitung durch Funkensprung (Lichtbogen). Als Stromquelle eignen sich beispielsweise solche Quellen wie Elektrokauter, VAPR (Produktename, Johnson&Johnson) oder Mikrowellensender.

Im Falle von radiofrequenter Wechselspannung ist die bevorzugte Frequenz der Wechselspannung: > 20 kHz, typischerweise > 300 kHz bis 3 MHz (Radiofrequenz). Typische durchschnittliche Leistung der Stromquelle: Für kleine Pins oder Fixationselemente (Durchmesser 0.1 - 5.0 mm): ca. 0.1 - 50.0 Watt, vorzugsweise 0.5 -10.0 Watt. Für die Fixation von grossen Prothesen oder für das Füllen grosser Knochendefekte 1-2'000 Watt.
Die Spitzenleistung während einzeln applizierter Pulse kann 5kW und mehr erreichen. Typische elektrische Spannung: 20 Volt- 3000 Volt, bevorzugt 20 - 300 Volt.
Typische Stromstärken: 0.01-100.00 Ampere, bevorzugt 0.05-10.00 Ampere.
Form der Wechselspannung (radiofrequenter Wechselstrom): Sinusförmig, rechteckig, trapezoid etc, asymmetrisch oder symmetrisch, gepulst oder kontinuierlich.
Typische Pulslängen: 0.1 ms - 5.0 ms.

Insbesondere ist zu berücksichtigen, dass die Stromstärke geregelt werden kann, durch Messung des Widerstands/Impedanz des Stromkreises (Ohm), des Stromflusses (Ampere), der abgegebenen Leistung (Watt) oder durch eine direkte (z.B. Wärme-Sensor) oder indirekte (z.B. Infrarotkamera), Messung der Wärme des Implantats oder des umgebenden Gewebes. So kann eine übermässige Erwärmung verhindert werden und Implantat wie auch Gewebe oder andere Implantate (Faden) geschont werden. Häufig verfügen die Elektrokaustikgeräte bereits über einen solchen Regelmechanis-mus welcher dafür auch verwendet werden kann (zur konstanten oder modulierten Leistungsabgabe). Eine andere Möglichkeit besteht darin, den mechanischen Widerstand des Implantats beim verformen zu messen und die elektrische Leistungsabgabe entsprechend zu regulieren. Als weiterer regelnder Effekt kann eine Widerstandsänderung des Polymers bei Erwärmung oder Erweichung verwendet werden, wodurch die Erwärmung kontrolliert werden kann (siehe oben im Text).

Die biokompatiblen und biodegradierbaren Polymere für das erfindungsgemässe medizinische Implantat können aus der folgenden Gruppe gewählt werden: Poly-alpha-hydroxyester, Polyorthoester, Polyanhydrid, Polyphosphazenes, Poly(propylenfumarat), Polyesteramid, Polyethylenfumarat, Polylaktid, Polyglykolid, Polycaprolacton, Trimethylencarbonat, Polydioxanon, Polyhydroxybutyrat, sowie deren Copolymere und Gemische.

Die biokompatiblen, nicht biodegradierbaren thermoplastischen Polymere für das erfindungsgemässe medizinische Implantat können aus der folgenden Gruppe gewählt werden: Polyethylen, Polystyrol, Polyester, Polypropylen, Polysulfon.

Die thermoplastischen Polymere für das erfindungsgemässe medizinische Implantat können beispielsweise aus der folgenden Gruppe der Stoffe ausgewählt werden, wobei je nach Indikation mit Zusätzen der Erweichungspunkt reduziert werden muss:
Acrylonitril-butadien-styrol (ABS), Polyacryl, Celluloid, Celluloseacetat, Ethylenvinylacetat (EVA), Ethylenvinylalkohol (EVAL), Fluoroplastic , Ionomere, Polyakrylat, Polyacrylonitril (PAN oder Acrylonitril), Polyamid (PA oder Nylon), Polyamidimide (PAI), Polyaryletherketone (PAEK oder Ketone), Polybutadien (PBD), Polybutylen (PB), Polybutylenterephthalat (PBT), Polyethylenterephthalat (PET), Polycyclohexylendimethylenterephthalat (PCT), Polycarbonate (PC), Polyketone (PK), Polyester, Polyethylen (PE), Polyetheretherketone (PEEK), Polyetherimid (PEI), Polyethersulfon (PES), Polyethylenechlorinat (PEC), Polyimid (PI), Polymethylpenten (PMP), Polyphenyleneoxid (PPO), Polyphenylenesulfid (PPS), Polyphthalamid (PPA), Polypropylen (PP), Polyurethan (PUR), Polysulfon (PSU), Polyhydroxyethylmethacrylat (PHEMA).

Die gewünschte Thermostabilität einzelner Zonen des medizinischen Implantates kann durch die Wahl der Materialien nach Leitfähigkeit, Schmelzpunkt sowie spezifischem elektrischen Widerstand der einzelnen Materialien variiert werden.

Falls das thermoplastische Material, welches für das erfindungsgemässe medizinische Implantat vorgesehen ist selbst nicht überall leitend ist, kann es durch Inkorporation von geeignete elektrisch leitenden Elementen (z.B. Kabel, elektrische Leitungen, Kernen aus Stahl oder Titan) zumindest teilweise zu einem elektrisch leitfähigen Thermoplasten modifiziert werden und somit als stromzuführende Elektrode ausgelegt werden. Dabei kann die Kontaktzone, welche mit dem Körper des Patienten in elektrischen Kontakt treten soll, voll oder teilweise mit leitfähigem Thermoplast beschichtet werden. Wenn die Kontaktzone nur teilweise mit leitfähigem Thermoplast beschichtet ist, dann sollte die übrige Oberfläche vorzugsweise nicht elektrisch leitend sein. Dies kann durch geeignete Materialwahl oder durch eine geeignete Beschichtung erfolgen, wie z.B. Hydroxyapatit oder anderen, beispielsweise osteokonduktiven, osteoinduktiven oder osteogenen Materialien.

Im Folgenden werden die Verfahrensschritte bei der Verwendung eines erfindungsgemässen medizinisches Implantates näher beschrieben:
a) Vorbereitung des Knochens. Z.B. Einbringen eines Bohrlochs;
b) Ansetzen des Fixationselementes in das Bohrloch;
c) Erwärmen des (thermoplastischen) Polymers des Implantats.
d) Eindrücken des Implantats in das zu fixierende Gewebe;
e) Abkühlen lassen des Implantats, was z.B. mittels aktiver Kühlung unterstützt werden kann.

In einer bevorzugten Ausführungsform ist das Polymer derart ausgewählt, dass die Erweichung unterhalb einer Erwärmungstemperatur von 250°C stattfindet.

In einer weiteren Ausführungsform findet die Erweichung unterhalb einer Erwärmungstemperatur von 150°C, vorzugsweise unterhalb von 100°C statt. Der Vorteil dieser Ausführungsform liegt darin, dass eine gewebeschonende Implantation im Körper (Mensch oder Tier) ermöglicht wird.

In wiederum einer weiteren Ausführungsform sind ausser dem Polymer selbst keine anderen Bauteile des Implantats für die Erwärmung des Implantats vorgesehen. Diese Ausführungsform zeichnet sich durch eine erhöhte Einfachheit in der Fabrikation und Applikation des Implantates aus.

In einer anderen Ausführungsform umfasst das medizinische Implantat Mittel zur Befestigung einer Elektrode.

In wiederum einer anderen Ausführungsform bestehen die Mittel aus einer Vertiefung in oder einer Erhebung an der Oberfläche des Polymers.

In einer weiteren Ausführungsform bestehen die Mittel aus einem Material mit einem elektrischen spezifischen Widerstand ρ_{M} < ρ. Der Vorteil dieser Ausführungsform liegt darin, dass dadurch der elektrische Strom bevorzugt durch das Polymer fliesst und nicht durch die Mittel zur Befestigung der Elektrode, so dass letztere nicht mitschmelzen.

In einer anderen Ausführungsform ist das Polymer ein Halbleiter, vorzugsweise eine organischer Halbleiter.

In wiederum einer anderen Ausführungsform umfasst das Polymer Molekülketten mit ausgedehnten konjugierten Doppelbindungen.

In wiederum einer weiteren Ausführungsform ist der elektrische spezifische Widerstand ρ grösser als 500 Ohm·cm, vorzugsweise grösser als 1500 Ohm·cm.

In einer anderen Ausführungsform ist der elektrische spezifische Widerstand ρ grösser als 3'000 Ohm·cm, vorzugsweise grösser als 10'000 Ohm·cm.

In wiederum einer anderen Ausführungsform weist das Polymer einen Oberflächenwiderstand von mindestens 10⁻¹ Ohm/square, vorzugsweise mindestens mindestens 10² Ohm/square auf.

In einer weiteren Ausführungsform weist das Polymer einen Oberflächenwiderstand von höchstens 10¹² Ohm/square, vorzugsweise höchstens 10⁷ Ohm/square auf.

In wiederum einer weiteren Ausführungsform weist das Polymer eine Volumenleitfähigkeit von mindestens 10⁻¹¹ S/m, vorzugsweise mindestens 10⁻⁴ S/m auf.

In einer anderen Ausführungsform weist das Polymer eine Volumenleitfähigkeit von höchstens 10¹ S/m, vorzugsweise höchstens 10° S/m, auf. Typischerweise beträgt die Volumenleitfähigkeit höchstens 0,1 S/m.

In wiederum einer anderen Ausführungsform wird der elektrische Widerstand im Implantat durch das Schmelzen des Polymers oder die Erwärmung des Implantates reduziert.

In einer weiteren Ausführungsform wird der elektrische Widerstand im geschmolzenen oder erwärmten Zustand des Implantates mindestens um einen Faktor 0.5, vorzugsweise um einen Faktor 10 reduziert.

In einer anderen Ausführungsform wird der elektrische Widerstand im geschmolzenen oder erwärmten Zustand des Implantates um einen Faktor >100 reduziert. Der Vorteil dieser Ausführungsform liegt darin, dass sich dadurch bereits geschmolzene Areale nicht weiter aufheizen und das umgebende Gewebe schonen.

In wiederum einer weiteren Ausführungsform wird der elektrische Widerstand im Implantat durch das Schmelzen des Polymers oder die Erwärmung des Implantates erhöht.

In wiederum einer anderen Ausführungsform wird der elektrische Widerstand im geschmolzenen oder erwärmten Zustand des Implantates mindestens um einen Faktor 0.5, vorzugsweise um einen Faktor 10 erhöht.

In einer weiteren Ausführungsform ist das Polymer isotrop.

In einer anderen Ausführungsform ist das Polymer anisotrop.

In wiederum einer weiteren Ausführungsform ist das Polymer ein thermoplastisches Material.

In wiederum einer anderen Ausführungsform wird das thermoplastische Material aus der Gruppe der Polyacetylene, Polyaniline, Poly(ethylenedioxythiophene), Poly(phenylenvinylene), Polyarylene, Polyspiro-bifluorene, Polydialkylfluorene, Polythiophene oder Polypyrrole genommen.

In einer weiteren Ausführungsform ist das thermoplastische Material aus folgenden Gruppen ausgewählt:
- thermoplastische Polymere, die selbst elektrisch leitend sind;
- Gemische der nicht elektrisch leitenden thermoplastischen Polymere (Matrix) mit Füllern oder Zusätzen, welche die Leitfähigkeit ermöglichen;
- Copolymere, welche aus elektrisch leitenden und elektrisch nicht leitenden Polymeren zusammengesetzt sind;
- Leitfähige Polymere, bei denen Elektrizitätsanwendung oder Erwärmung eine chemische Reaktion (z.B. Polymerisierung) bzw. physikalische Reaktion (z.B. Änderung der Geometrie) hervorrufen kann.
- Kombinationen der obenerwähnten Materialien.

In wiederum einer weiteren Ausführungsform umfasst das medinische Implantat neben dem Polymer noch Implantatteile aus weiteren Materialien, vorzugsweise aus folgenden Gruppen ausgewählt: Metalle, Carbon, Keramik, PEEK, nicht thermoplastische Polymere, welche vorzugsweise aus der Gruppe der Polymethylmetacrylate ausgewählt werden und/oder anorganische Materialien, so wie Kalziumphosphat, Kalziumsulfat oder Knochenzement.

In einer anderen Ausführungsform ist das Polymer als solches selbst elektrisch leitend.

In wiederum einer anderen Ausführungsform wird die elektrisch Leitfähigkeit des Polymers durch geeignete Dotierung erreicht.

In einer weiteren Ausführungsform ist das Polymer mit einer elektrisch leitfähigen Keramik, insbesondere einer glasartigen oder amorphen Struktur kombiniert.

In wiederum einer weiteren Ausführungsform wird das Polymer durch elektrisch leitende Zusatzstoffe zu einem elektrisch nichtleitenden Polymer erhalten. Geeignete Zusatzstoffe sind beispielsweise: Russpartikel ("carbon black"), vorzugsweise 3-50%; Kohlefasern mit einer Länge von höchstens 1 mm , vorzugsweise 3-50% möglichst homogen im Polymer verteilt; Kohle-Nanotubes, vorzugsweise 0.1-5%; Metallpartikel, insbesondere Eisen, Titan, Gold, Magnesium, Stahl; Salze, insbesondere NaCl, Barium, Magnesiumsalze; Proteine, Knochenmaterial; Oele; Silikate. Alle leitenden Zusatzstoffe können in Kugelform, Flocken, usw. zugegeben werden.

In einer anderen Ausführungsform bestehen die elektrisch leitenden Zusatzstoffe aus jedem möglichen elektrisch leitenden festen oder flüssigen Material in Form von Partikeln, Körnchen, Partikelanhäufungen irgendeiner äusseren Form.

In wiederum einer anderen Ausführungsform werden die elektrisch leitenden Zusatzstoffe aus folgenden Materialien gewählt:
- Metallische Materialien z.B. Eisen, Magnesium, Gold, Silber, Legierungen oder Amalgame;
- Carbonpartikel z.B. Ruß, Carbon Nanoröhren (carbon nanotubes), Fullerene;
- Salze oder Substanzen, die Wasser in einer schnellen Weise anziehen können, um den Fluss von Elektrizität in der erforderlichen Menge zuzulassen. Solche Salze können aus z.B. Natriumchlorid, Natrium Sulfat oder Magnesiumsalzen bestehen.
- Elektrisch leitende Polymere z.B. Polymere der Pyrrole, Anilline, Dialkylfluore, Tthiophene oder Eethylenedioxythiophene gewählt werden.
- biocompatible Öle, z.B. Silikone;
- wässerige Lösungen, vorzugsweise Salzlösungen.

Die Menge der Füller/Zusätze muss dem beabsichtigten Gebrauch angepasst werden, so dass die elektrische Leitfähigkeit an den gewünschten Zweck angepasst wird, z.B. Anpassen des elektrischen Widerstands des Materials so, dass das geheizte Material thermoplastisch, fließfähig oder sogar flüssig wird, bzw. so dass beim Einleiten einer bestimmten chemischen Reaktion das Material sich verhärtet.

In einer weiteren Ausführungsform weist das Polymer eine offenporige Struktur auf. Der Vorteil dieser Ausführungsform liegt darin, dass dies das Einheilen von Knochen fördert und das Halten von elektrisch leitenden Flüssigkeiten, Gelen oder anderen Stoffen erlaubt.

In wiederum einer weiteren Ausführungsform weist das Polymer kapillare Kanäle auf. Damit ist der Vorteil erreichbar, dass das Eindringen von Salzlösung aus dem Körper oder anderen Flüssigkeiten erlaubt wird, um die Leitfähigkeit zu modulieren.

In einer anderen Ausführungsform weist das Polymer hydrophile Eigenschaften auf.

In wiederum einer anderen Ausführungsform besteht das medizinische Implantat aus einem homogenen Material.

In einer weiteren Ausführungsform weist das homogene Material keine innere Struktur auf.

In wiederum einer weiteren Ausführungsform liegt das Polymer in Form einer Implantatbeschichtung vor.

In einer anderen Ausführungsform ist nur ein Teil der Oberfläche des Implantates mit dem Polymer beschichtet.

In wiederum einer anderen Ausführungsform umfasst das Polymer Zonen mit einem unterschiedlichen elektrischen spezifischen Widerstand p, insbesondere in Form von Oberflächenbeschichtungen.

In einer weiteren Ausführungsform weist die Beschichtung eine variable Schichtdicke auf.

In wiederum einer weiteren Ausführungsform ist das gesamte Implantat oder nur das Polymer teilweise mit nicht elektrisch leitenden Materialien beschichtet. Damit ist der Vorteil erreichbar, dass sich durch diese Ausführung der Beschichtung ein Pfad für den Stromdurchfluss bestimmen lässt. Die nicht leitende Schicht soll als Isolation dienen und einen Kurzschluss verhindern.

In einer anderen Ausführungsform weist das nicht elektrisch leitenden Material osteokonduktive, osteoinduktive oder osteogene Eigenschaften auf.

In wiederum einer anderen Ausführungsform ist das nicht elektrisch leitende Material ein Polylactid oder Hydroxyapatit.

In einer anderen Ausführungsform umfasst das Polymer eine Mischung aus mindestens zwei verschiedenen körperverträglichen, elektrisch leitenden thermoplastischen Materialien. Diese Ausführungsform zeichnet sich durch Zonen mit verschiedenen Widerständen mit konstanter Form des Implantates aus. Das elektrisch leitende, thermoplastische Material kann in Form von Polymer, Gel, Paste, Wachs vorliegen.

In wiederum einer anderen Ausführungsform weist das medizinische Implantat eine feste Form auf. Der Vorteil dieser Ausführungsform liegt darin, dass auf das Implantat besser eine äussere Kraft aufgebracht werden kann.

In einer weiteren Ausführungsform liegt das Polymer in Granulatform vor. Damit ist der Vorteil erreichbar, dass das Polymer auf diese Weise in Zwischenräume, Lücken oder Hohlräume eingefüllt und dort verfestigt werden kann.

In wiederum einer weiteren Ausführungsform ist das medizinische Implantat aus Fasern hergestellt, wobei das Polymer vorzugsweise als Beschichtung der Fasern dient. Die Fasern können geflochten, gewoben oder gezwirnt sein und als einzelne Fäden, Netz, Tuch oder Tasche vorliegen. Der Vorteil dieser Ausführungsform liegt darin, dass das textile/fasrige Implantat so in die gewünschte Form gebracht werden kann und durch Stromfluss verfestigt/verklebt werden kann.

In einer anderen Ausführungsform liegt das medizinische Implantat in Form eines offenporigen Schaums oder Schwammes vor.

In einer weiteren Ausführungsform ist das medizinische Implantat als Knochenfixationselement ausgebildet, vorzugsweise in Form einer Knochenschraube, Knochenstift, Knochendübel, Pin, Platte, Dübel, Schlauch (Rohr), Faden, Faden in Schlauch/Rohr oder Anker (mit Fadengleitöse).

In wiederum einer weiteren Ausführungsform ist das Polymer stabförmig ausgebildet und weist ein zentrales Längsloch auf, welches zur längsverschieblichen Aufnahme eines mit einer Elektrode verbindbaren Metallstiftes oder eines fest an einer Elektrode angebrachten Stiftes geeignet ist.

In einer anderen Ausführungsform umfasst das medizinische Implantat einen im Längsloch aufnehmbaren Metallstift oder Metalldraht, welcher bis auf einen endständigen Teilabschnitt mit einer Isolation versehen ist.

In wiederum einer anderen Ausführungsform ist das Polymer stabförmig ausgebildet und umfasst eine periphere, nicht elektrisch leitende Isolationsschicht.

In wiederum einer weiteren Ausführungsform ist das Polymer stabförmig ausgebildet und umfasst eine äussere Hülse aus einem zweiten leitenden Polymer mit höherem Widerstand.

In einer anderen Ausführungsform ist das Polymer als Perle ausgebildet ist und mit einer Elektrode in der Form eines Drahtes lösbar verbindbar.

In einer weiteren Ausführungsform ist das medizinische Implantat als Zahnimplantat oder Zahnwurzelimplantat ausgebildet.

In wiederum einer weiteren Ausführungsform befindet sich das Polymer mindestens zum Teil in einem erweichten Zustand.

In einer anderen Ausführungsform ist der erweichte Zustand durch einen das Polymer durchfliessenden elektrischen Strom erzeugt.

In wiederum einer anderen Ausführungsform ist der elektrischen Strom durch eine externe Stromquelle erzeugt.

In einer weiteren Ausführungsform ist die Stromquelle eine Wechselstromquelle.

In wiederum einer weiteren Ausführungsform ist das Polymer mit einem Wechselstrom der Frequenz v höher als 20'000 Hz, vorzugsweise höher als 300'000 Hz erwärmbar und erweichbar.

In einer anderen Ausführungsform ist das Polymer mit einem Wechselstrom der Stromstärke I zwischen 0,001 bis 10 Ampère erwärmbar und erweichbar.

In wiederum einer anderen Ausführungsform ist das Polymer mit einem Wechselstrom der Spannung U zwischen 20 bis 300 Volt erwärmbar und erweichbar.

In einer weiteren Ausführungsform ist das Polymer mit einem zu erweichenden Volumen V mit einem Wechselstrom der Leistungsdichte P = 0.005 - 5 Watt/mm³ innert ca. 0.1 -10 Sekunden erwärmbar und erweichbar. Die so applizierte Energie E entspricht dabei ca. E = 0.0005 - 50 Watt*Sekunden/mm³.

In wiederum einer weiteren Ausführungsform weist das Polymer keine gleichmässige Leitfähigkeit auf, und letztere ist vorzugsweise an der Oberfläche des Implantats geringer als im Inneren des Implantats. In beiden Ausführungsformen (bipolar und unipolar) kann das erfindungsgemässe Implantat gegen aussen Zonen von elektrischer Isolation aufweisen, d.h. es kann bei einem unipolar verwendeten Pin, z.B. der elektrisch leitende Schaft mit einer nichtleitenden Schicht isoliert sein und nur z.B. die Spitze des Implantats elektrisch Kontakt mit dem Körper erhalten. So kann erreicht werden, dass das Implantat,z.B. zuerst an der Spitze aufweicht und so in den Knochen geschmolzen werden kann, während der Schaft des Pin seine Stabilität beibehält. Damit ist der Vorteil erreichbar, dass eine selektive Erwärmung des Polymers möglich ist und zwar gezielt dort, wo zweckmässigerweise durch den Stromfluss aufschmelzen, sich verflüssigen oder erweichen erfolgen soll, nämlich vorzugsweise an der Oberfläche des Implantats, welche mit dem Gewebe des Patienten in Kontakt steht.

In einer anderen Ausführungsform umfasst das elektrisch leitfähige Polymer des medizinischen Implantates keine internen Bauelemente, Strukturen oder Fasern, welche von aussen mit elektrischer Energie beaufschlagt werden und sich dadurch erwärmen.

In wiederum einer anderen Ausführungsform erfolgt die Wärmeerzeugung im elektrisch leitfähigen Polymer des medizinischen Implantates allein durch Stromdurchfluss durch das elektrisch leitfähige Polymer.

In einer weiteren Ausführungsform ist das gesamte elektrisch leitfähige Polymer des medizinischen Implantates so stromdurchflossen, dass eine homogene Erwärmung desselben erfolgt.

In wiederum einer weiteren Ausführungsform ist das gesamte elektrisch leitfähige Polymer des medizinischen Implantates so stromdurchflossen, dass eine inhomogene Erwärmung desselben erfolgt.

In einer möglichen Anwendung wird der Körper des Patienten selbst als neutrale Elektrode des Stromkreises verwendet.

In einer weiteren möglichen Anwendung ist das medizinische Implantat im Stromkreis zwischen zwei Knochen geschaltet. Dies eignet sich für eine Knochen-Thermoplast-Knochen Anwendung des medizinischen Implantates.

In wiederum einer weiteren möglichen Anwendung ist eine Elektrode des Stromkreises mit einem Knochenfragment und die zweite Elektrode mit dem zugehörigen Knochen oder sonst mit dem Körper des Patienten verbunden.

In einer anderen möglichen Anwendung ist eine Elektrode des Stromkreises mit einem Knochenfragment und die zweite Elektrode mit dem zwischen Knochenfragment und Knochen eingeführten medizinischen Implantat verbunden.

In wiederum einer anderen möglichen Anwendung ist die Implantationsstelle eine Knochenbohrung.

In einer weiteren möglichen Anwendung ist das medizinische Implantat im nicht erweichten Zustand gegenüber der Knochenbohrung überdimensioniert.

In wiederum einer weiteren möglichen Anwendung ist das medizinische Implantat im nicht erweichten Zustand gegenüber der Knochenbohrung nicht überdimensioniert und weist eine innere Vorspannung auf. Das Aufbringen einer Vorspannung kann z.B. bei der Herstellung, z.B. durch Spritzguss erfolgen.

In einer anderen möglichen Anwendung wird das elektrisch leitfähige Polymer in Form einer Stange durch einen isolierten Hohlraum eines Implantates eingeführt und umfasst vorzugsweise einen elektrisch leitenden Kern.

In wiederum einer anderen möglichen Anwendung wird das elektrisch leitfähige Polymer in ein einen Hohlraum mit radialen Austrittslöchern aufweisendes Implantat eingeführt.

In einer weiteren möglichen Anwendung erfolgt Verwendung des medizinischen Implantates für die Vertebroplastik.

In wiederum einer weiteren möglichen Anwendung erfolgt Verwendung des medizinischen Implantates für die Verriegelung und/oder Zentrierung von Implantaten, insbesondere Marknägeln nach deren Einbringung in den Knochen.

In wiederum einer anderen Ausführungsform ist das Polymer derart ausgewählt, dass die Erweichung oberhalb einer Erwärmungstemperatur von 40°C stattfindet.

### Beispiel 1 (Plattenosteosynthese)

Eine resorbierbare Osteosyntheseplatte mit einer Dicke von 1 mm aus einem Poly-D,L-Lactid wurde auf die zu fixierenden Knochenfragmente aufgebracht und die benötigten Löcher in den Knochen gebohrt. In diesem Beispiel handelte es sich um eine Platte mit Löchern für 2.0 mm Schrauben. Es wurden Löcher von 1.7 mm in den Knochen gebohrt. Anschliessend wurde ein elektrisch leitender Pin mit einem 2.0 mm Durchmesser auf eine Elektrode aufgesetzt, welche an einen handelsüblichen Elektrokauter angeschlossen war. Der Pin bestand aus Poly-D,L-Lactid, welchem 15 Prozent Carbon Black beigemischt wurde.
Der Patient wurde auf konventionelle Weise an die neutrale Elektrode des Elektrokauters angeschlossen. Der Pin wurde durch das Schraubenloch in der Platte auf des vorgebohrte Loch aufgesetzt und unter Strom gesetzt (Leistung 5 Watt). Der Strom floss durch den elektrisch leitenden Pin und erwärmte diesen. Da der grösste elektrische Spannungsabfall am Übergang Knochen zu Pin bestand, entstand hier im Pin die grösste Wärme, wodurch der Pin vor allem an seiner Oberfläche aufweicht wurde. Durch sanften Druck auf die Elektrode konnte nun der Pin in das in den Knochen vorgebohrte Loch geschoben werden und das thermoplastische Material floss in die zugänglichen intertrabekulären Zwischenräume im spongiösen Knochen. Nach Ausschalten des Stroms kühlte das Polymer wieder ab und erstarrt in weniger als 1 Minute aus. Der mit einem etwas überdimensionierten (d.h. breiter als die Plattenbohrung) Kopf versehen Pin hielt nun die Platte an der gewünschten Stelle. Nach Ausschalten des Strom kühlte das Polymer wieder ab und härtete innert kurzer Zeit (< 1 Minute) aus. Der mit einem etwas überdimensionierten (d.h. breiter als die Plattenbohrung) Kopf versehene Pin hielt nun die Platte an der gewünschten Stelle.

### Beispiel 2 (Plattenosteosynthese)

Bei einer Variante des Beispiels 1 wurde eine Knochenplatte verwendet, welche ebenfalls aus dem gleichen elektrisch leitfähigen Thermoplasten wie der oben beschriebene Pin gefertigt wurde. Der Pin wurde analog zum obigen Beispiel eingebracht. Sobald der Kopf des Pins in Kontakt mit der Platte geriet fand hier ebenfalls eine Verschmelzung zwischen Platte und Pin statt da die Platte im Bereiche des Loches ebenfalls elektrisch leitfähig ist und dort ein Verschmelzen von Platte und Kopf erreicht wurde. Nach dem Abkühlen waren Pin und Platte fest miteinander verbunden, die Verbindung wurde dadurch winkelstabil.

### Beispiel 3 (Knochenanker)

Die zu lösende Aufgabe war hier die Fixation eines Fadens im Knochen, um mit dem Faden z.B. eine Sehne oder ein anderes Knochenstück zu halten. Dafür wurde ein Loch mit einem Durchmesser von 3 mm bis 15 mm tief in den Knochen gebohrt. In das Loch im Knochen wurde ein Faden eingeführt, welcher einen hohen Schmelzpunkt aufweist. Auf das Loch wurde danach ein Anker aufgesetzt welcher etwas dicker als das Loch war. Der Anker wurde aus Polypyrrol gefertigt, welches eine Leitfähigkeit von 1000 Ohm/square aufwies.
Analog zum Beispiel 1 wurde auch hier der Anker mittels Elektrokauter unter Strom gesetzt und nach Aufweichen durch den Stromfluss in den Knochen gedrückt. Nach Ausschalten des Stroms, erstarrte das Polymer und der Anker hielt im Knochen und mit ihm der Faden.

### Beispiel 4 (Knochenanker)

Bei einer Abwandlung des Beispiels 3 wurde der Faden durch eine Querbohrung im Anker geführt, der Anker anschliessend in den Knochen geführt und dort mit der Elektrode gehalten. Anschliessend wurde die abgerissene Sehne mittels des Faden befestigt. Hierbei wurde der Faden unter einer Zugkraft gehalten. Durch das gleichzeitig erfolgte Einschalten des Stroms schmolz der Anker teilweise auf und verklebte unter etwas Druck mit dem Faden und gewann so halt im Knochen. Nach dem Abkühlen innert ca. 30Sekunden konnte dann die Zugkraft auf dem Faden gelöst werden. Das sonst notwendige Verknoten des Fadens erübrigte sich.

### Beispiel 5 (Implantation einer Prothese)

Bei einem Zahnimplantat aus Titan wurde das distale Drittel mit einem leitfähigem Thermoplast umgeben. Hierzu wurde das Implantat mehrfach eine Lösung aus Poly-D,L-lactid mit 25% Carbon Black getaucht und zwischen den Tauchgängen getrocknet. Die oberen zwei Drittel der Oberfläche wurden aus Isolationsgründen mit einem schnell sich lösenden Polylactid-co-glykolid mit geringem Molekulargewicht in analoger Weise beschichtet.
Die von der Wurzelspitze weggewandte Seite wurde an eine Stromquelle angeschlossen. Das Implantat wurde in das mit Untermass vorgebohrte Loch gesetzt und der Strom eingeschalten. Sobald der Strom durch die Elektrode in das Implantat, dann durch das Polymer und wieder durch den Knochen wegfloss, erweichte die Beschichtung am distalen Ende und das Implantat konnte nun mittels Druck in die Tiefe des Lochs gedrückt werden. Das Erstarren des Polymers im Knochen führte zu einer primären, belastungsstabilen Verbindung zwischen Knochen und Implantat. Die Beschichtung aus Polylactid-co-glykolid degradiert innert weniger Tagen und erlaubt danach ein Anwachsen von Knochen an das Titanimplantat.

### Beispiel 6 (Gefässclip)

Der Clip diente zum Abklemmen von Blutgefässen zur Blutstillung. Er bestand im Wesentlichen aus zwei Schenkeln und einem Scharnier. Die Schenkel wurde mit einer bipolaren Klemme gegriffen und das Blutgefäss dazwischen gehalten. Die Schenkel wurden unter Strom gesetzt und zusammengedrückt. Durch den Stromfluss erweichte das Scharnier und erlaube ein Verbiegen des Clips. Beim Aufeinandertreffen der dem Scharnier abgewandten Enden der Schenkel floss auch hier ein Strom und führte zu einem Aufschmelzen und gewünschten Verbinden der beiden Schenkel.

### Beispiel 7 (Fadenclip)

Dieselbe Anwendung eines Clips wie in Beispiel 7 beschrieben konnte auch für die Fixation von Fäden verwendet werden zur Vermeidung von Knoten. Der Clip wies ein Längsmass von 7mm auf, bestehend aus zwei ebenso langen Armen. Der Querdurchmesser der Arme betrug 3x3mm.

### Beispiel 8 (Vertebroplastik)

Bei einer Patientin mit einer osteoporotischen Kompressionsfraktur des ersten Lendenwirbels wurde durch die Pedikel (in Lokalanästhesie) von dorsal ein Loch von 4mm Durchmesser in den Wirbelkörper gebohrt (Länge ca. 4cm). Durch das Loch wurde von dorsal noch ohne Stromanwendung ein Poly-D-L-lactid Pin eingeführt (Durchmesser 3.9mm), versetzt mit Polypyrrol und einer dadurch resultierenden Leitfähigkeit von 1200ohm/square. Der Pin selbst war aussen mit einer 0.5mm dicken Isolationsschicht aus Poly-D-L-lactid überzogen und wies ein zentrales Längsloch von 0.6mm Durchmesser auf. In diesem Längsloch findet sich ein mit der Elektrode verbundener Metallstift (chirurgischer Stahl) von 0.55mm Durchmesser. Nun wurde die Elektrode eingeschaltet und der Pin in den Wirbelkörper geschoben. Da der Pin an der Spitze keine Isolation aufwies machte er dort Kontakt mit dem Knochen und schmolz auf. Unter nachschieben des Pins (bei Halten der Position der Elektrode im Zentrum, d.h. der Pin wird wie ein dickwandiges Rohr auf der Elektrode in die Tiefe geschoben) konnte so eine Füllung des Wirbelkörpers mit dem Poly-D-L-lactid erreicht werden. Der Pin verlor beim Aufschmelzen kontinuierlich seine Isolation an der Spitze, sodass unter kontinuierlichem Schmelzen des Materials des Pins in den Wirbelkörper nachgeschoben werden konnte. Nach 2 Minuten auskühlen war der Wirbelkörper belastungsstabil und schmerzfrei.

### Beispiel 9 (Elektrodendesign)

Hier beschreiben wir eine besonders günstige Anordnung von Implantat und Elektrode: Die Elektrode wird so ausgelegt, dass sie, analog zu Beispiel 9 nahe an den Ort geführt werden kann, wo der Strom appliziert werden soll. Allerdings hat in diesem Beispiel die Elektrode eine Isolation und ist nur an ihrer Spitze auf einer Länge von 7mm zirkumferentiell leitfähig. Analog zum Beispiel 9 wurde die Elektrode durch einen hohlen Pin (aus Polylactid mit Carbon Black 15%) geführt und durch den Pedikel in den Wirbelkörper geschoben. Nun konnte über die Elektrode der hohle Pin mit wenig Widerstand in den Wirbelkörper geschoben werden. Im Gegensatz zu Beispiel 9 ist hier der Pin nicht gegen die Pedikelwand isoliert und schmilzt dennoch nur an seiner Spitze, da die Elektrode nur dort Strom abgibt. Das klinische Resultat ist dasselbe wie in Beispiel 9. In einer erweiterten Ausführungsform war es möglich, die Spitze der Elektrode mit einem Wärmesensor zu versehen, um die entstandene Hitze zu messen und mittels Regelungsschaltung an der Stromquelle zu regulieren. So konnte eine übermässige Hitzeentwicklung zusätzlich vermieden werden.

### Beispiel 10 (Defektfüllung)

Derselbe Pin wie in Beispiel 9 beschrieben wurde auch zum Füllen des Defektes von Knochen verwendet, hier zum Füllen eines Tibiakopfdefekts. Dafür wurde bei einem Patienten mit Tibiakopffraktur ein 4mm Loch von ventral durch die Kortikalis zum Defekt hin gebohrt. (Länge 2cm). Durch dieses Loch wurde darauf der Pin in die Markhöhle und den spongiösen Raum des Knochens unter Stromanwendung geschoben, und so wie bei einer Verbundosteosynthese ein stabiler Knochen geschaffen. Die danach in dieses Gebiet eingebrachten Schrauben hielten im geschmolzenen Polymer vorzüglich. Es hat sich erwiesen, dass das nachträgliche hineinschmelzen von Polymer bei liegendem Osteosynthesematerial oder liegenden Prothesen zu ähnlich stabilen Verhältnissen führt.

### Beispiel 12 (Verbundosteosynthese)

Im Rahmen einer Schenkelhalsfraktur bei Osteoporose wurde durch den Schenkelhals eine dynamische Hüftschraube implantiert, welche folgendermassen modifiziert war: innen mit einer zusätzlichen Längsbohrung von 3mm Durchmesser versehen und an der Spitze wo sich das Gewinde befindet mit 10 radiären Löchern von 1 mm Durchmesser versehen, welche eine Kommunikation von zentraler Bohrung und dem Knochen ermöglichen. In diese zentrale Bohrung wurde ein, wie in Beispiel 9 isolierter Pin von 2.9mm Durchmesser eingeführt und hinten unter Strom gesetzt. Unter Stromanwendung konnte so der Pin im Innern der Schraube geschmolzen werden und das verflüssigte Polymer drang durch die Löcher nach aussen in den Knochen und schuf so eine Augmentation des Knochens in welchem das Implantat hält. Nach aushärten des Polymers (2 Min.) war die Schraube belastungsstabil.

### Beispiel 13 (Stent)

Im Rahmen einer Gefässdilatation bringt der Radiologe durch einen femoralen Zugang einen Herzkatheter in die femoralen Gefässe ein und führt diesen Katheter zu einer verengten Nierenarterie. An der Spitzer des Katheters ist ein Ballon angebracht, um diesen ein zusammengefalteter Stent aus Polypyrrol (Durchmesser 1.5mm, Länge 2cm). Der Ballon selbst ist monopolar leitend und liegt zusammengefaltet im Stent. Nun wird der Ballon unter Strom gesetzt und der Strom fliesst durch den Stent und erwärmt und erweicht diesen. Nun kann der Ballon aufgeblasen werden und der Stent wird aufgedehnt bis ein adäquater Blutfluss erreicht wird. Der Strom wird ausgeschaltet und der Stent kühlt aus, härtet aus (innert 40 Sekunden) und hält das Gefäss offen.

### Beispiel 14 (Memoryeffekt)

Ein Knochenanker wird mit innerer Vorspannung mittels Spritzguss hergestellt (PLA/Polyanilin). In der nun vorhandenen erkalteten Form ist der Anker gerade (Länge 10mm, Durchmesser 3mm). Mit durch eine Oese im oberen Drittel des Ankers durchgefädelten Faden wird der Anker unter sanftem Druck in ein vorgebohrtes Loch am Aussenknöchel gestossen. Unter Wärmeeinwirkung durch monopolar aufgebrachten Strom wird die Relaxation des Ankers eingeleitet und er verbiegt sich. Dadurch verkeilt sich der Anker im Knochenloch und findet mechanischen Halt. Der Faden am Anker ist so nach 30 Sekunden belastbar und kann für eine Bankrekonstruktion verwendet werden.

### Beispiel 15 (Nagelverriegelung)

Ein Femurmarknagel wird zur Osteosynthese in den Femur eingebracht. Distal war bei dieser 86jährigen Patientin der Knochen aber zu weich für eine Verriegelung, daher hat der Operateur ein 4mm Loch von lateral durch die Kortikalis zum Nagel hin gebohrt. Ein 3.5mm Pin aus leitfähigem Kunststoff wurde durch das Loch zum Nagel geschoben. Nun wurde der Pin monopolar unter Strom gesetzt und in den Markkanal geschoben, wodurch er kontinuierlich am Nagel wegschmolz und den hohlen Markkanal auffüllte und dabei den Nagel einbettete. Damit sich das Implantatmaterial gut in der Markhöhle verteilte wurde eine relativ hohe Energie gewählt (70 Watt) und ein Polymer mit hoher Wärmekapazität gewählt, damit es sich nicht zu schnell abkühlt und erstarrt. Nach ausschalten des Stroms war der Nagel sicher im Zentrum des Femurs fixiert.

Die Erfindung und Weiterbildungen der Erfindung werden im Folgenden anhand der teilweise schematischen Darstellungen mehrerer Ausführungsbeispiele noch näher erläutert.

Es zeigen:
Fig. 1 einen Längsschnitt durch eine Ausführungsform des erfindungsgemässen medizinischen Implantates;
Fig. 2a einen Längsschnitt durch eine weitere Ausführungsform des erfindungsgemässen medizinischen Implantates;
Fig. 2b einen Längsschnitt durch wiederum eine weitere Ausführungsform des erfindungsgemässen medizinischen Implantates;
Fig. 3a einen Querschnitt durch eine andere, als Zahnimplantat ausgebildete Ausführungsform des erfindungsgemässen medizinischen Implantates vor dem Schmelzvorgang;
Fig. 3b einen Querschnitt durch die Ausführungsform gemäss Fig. 3a nach erfolgter Implantation;
Fig. 4 einen Längsschnitt durch eine weitere, als Hüftgelenkprothese ausgebildete Ausführungsform des erfindungsgemässen medizinischen Implantates nach erfolgter Implantation;
Fig. 5a eine Ansicht einer anderen Ausführungsform des erfindungsgemässen medizinischen Implantates;
Fig. 5b eine Ansicht der Ausführungsform gemäss Fig. 5a nach erfolgter Implantation;
Fig. 6a eine Ansicht einer weiteren Ausführungsform des erfindungsgemässen medizinischen Implantates;
Fig. 6b eine Ansicht der Ausführungsform gemäss Fig. 6a nach erfolgter Implantation;
Fig. 7a eine Ansicht einer anderen Ausführungsform des erfindungsgemässen medizinischen Implantates;
Fig. 7b eine Ansicht der Ausführungsform gemäss Fig. 7a nach erfolgter Implantation;
Fig. 8a einen Schnitt durch wiederum eine andere Ausführungsform des erfindungsgemässen medizinischen Implantates;
Fig. 8b einen Schnitt durch die Ausführungsform gemäss Fig. 8a nach erfolgter Implantation;
Fig. 9a einen Schnitt durch wiederum eine weitere Ausführungsform des erfindungsgemässen medizinischen Implantates;
Fig. 9b einen Schnitt durch die Ausführungsform gemäss Fig. 9a nach erfolgter Implantation;
Fig. 10 einen Schnitt durch wiederum eine weitere Ausführungsform des erfindungsgemässen medizinischen Implantates;
Fig. 11a einen Schnitt durch wiederum eine andere Ausführungsform des efindungsgemässen medizinischen Implantates;
Fig. 11b einen Schnitt durch die Ausführungsform gemäss Fig. 11a während Implantation;
Fig. 11c einen Schnitt durch die Ausführungsform gemäss den Fig. 11a und 11b nach erfolgter Implantation;
Fig. 12 einen Schnitt durch eine weitere Ausführungsform des efindungsgemässen medizinischen Implantates;
Fig. 13 einen Schnitt durch wiederum eine weitere Ausführungsform des erfindungsgemässen medizinischen Implantates;
Fig. 14 einen Schnitt durch eine andere Ausführungsform des erfindungsgemässen medizinischen Implantates;
Fig. 15a einen Schnitt durch wiederum eine andere Ausführungsform des erfindungsgemässen medizinischen Implantates; und
Fig. 15b einen Schnitt durch die Ausführungsform gemäss Fig. 15a nach erfolgter Implantation.

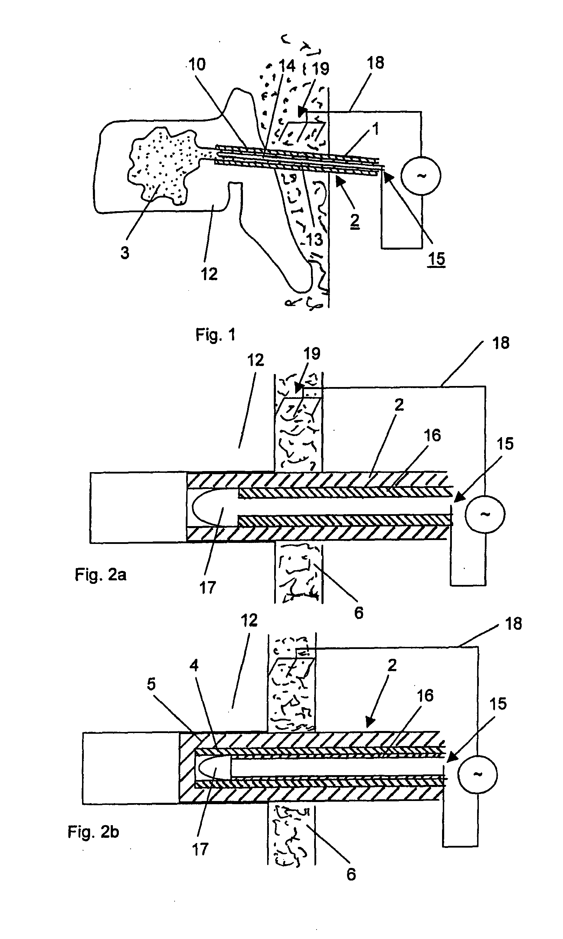

In der in Fig. 1 dargestellten Ausführungsform umfasst das medizinische Implantat einen Pin 2 mit einer peripheren Isolationsschicht 1 und wird für eine Anwendung in der Vertebroplastik verwendet (Beispiel 9). Durch ein vorgebohrtes Loch 10 in einem Pedikel eines zu behandelnden Wirbelkörpers 12 wird von dorsal noch ohne Stromanwendung ein Pin 2 aus einem Blend von Polypyrrol und Poly-D,L-lactid eingeführt.
Der Pin 2 selbst ist aussen mit einer 0.5 mm dicken Isolationsschicht 1 aus Poly-D-L-lactid überzogen und weist ein zentrales Längsloch 13 von 0.6mm Durchmesser auf. In diesem Längsloch 13 findet sich ein mit der Elektrode 15 verbundener Metallstift 14 (chirurgischer Stahl) von 0.55mm Durchmesser. Nach Einführung des Pins 2 wird der Strom eingeschaltet und der Pin 2 mit der angeschlossenen Elektrode 15 in den Wirbelkörper 12 geschoben. Da der Pin 2 an der Spitze keine Isolation aufweist macht er dort Kontakt mit dem Knochen und schmilzt auf. Unter Nachschieben des Pins 2 (bei Halten der Position der Elektrode 15 im Zentrum, d.h. der Pin 2 wird wie ein dickwandiges Rohr auf der Elektrode 15 in die Tiefe geschoben) kann so eine Füllung 3 des Wirbelkörpers 12 mit dem Poly-D-L-lactid erreicht werden. Nach 2 Minuten Auskühlen ist der Wirbelkörper belastungsstabil und schmerzfrei. Der Stromabfluss erfolgt "monopolar" durch den Körper des zu behandelnden Patienten via Nulleiter 18 (neutrale Elektrode oder Erdung) auf der Haut oder sonst wo am Körper des Patienten mittels einer grossflächigen Elektrode, wobei die Wechselspannung typischerweise via dem medizinischen Implantat eingespiesen wird. In einer anderen Ausführungsform kann der Pin 2 auch ohne Isolationsschicht 1 ausgeführt sein und durch ein in das Loch 10 eingeführtes, isolierendes Rohr oder isolierenden Schlauch geschoben werden.

Die in Fig. 2a dargestellte Ausführungsform unterscheidet sich von der in Fig. 1 dargestellten Ausführungsform nur durch eine andere Anordnung des medizinischen Implantates und der Elektrode 15, d.h. ein unterschiedliches Elektrodendesign (Beispiel 10). Die Elektrode 15 ist so ausgelegt, dass sie, analog zu Fig. 1 nahe an den Ort geführt werden kann, wo der Strom appliziert werden soll. Allerdings hat hier die Elektrode 15 eine Isolation 16 mit niedrigem Widerstand und ist nur an ihrer Spitze 17 auf einer Länge von 7mm zirkumferentiell leitfähig. Analog zur Ausführungsform in Fig. 1 wird die Elektrode 15 in den hohlen Pin 2 (aus Polylactid mit Carbon Black 15%) eingeführt und zusammen mit diesem durch den Pedikel in den Wirbelkörper 12 geschoben. Im Gegensatz zu Fig. 1 ist hier der Pin 2 nicht gegen die Pedikelwand isoliert und schmilzt dennoch nur an seiner Spitze, da die Elektrode 15 nur dort Strom überträgt. In einer erweiterten Ausführungsform (nicht gezeichnet) ist die Spitze 17 der Elektrode 15 mit einem Wärmesensor versehen, um die entstandene Hitze zu messen und mittels Regelungsschaltung an der Stromquelle zu regulieren. So kann eine übermässige Hitzeentwicklung zusätzlich vermieden werden. Die Stromübertragung erfolgt "monopolar" durch den Körper des zu behandelnden Patienten via Nulleiter 18 (neutrale Elektrode oder Erdung) auf der Haut 6 mittels einer grossflächigen Elektrode. Eine alternative Ausführungsform ist in Fig. 2b dargestellt, welche sich nur darin von Fig. 2a unterscheidet, dass der Pin 2 hier eine die Elektrode 15 umschliessende, innere Hülse 4 aus einem leitenden Polymer mit niederem Widerstand und koaxial eine äussere Hülse 5 aus einem leitendem Polymer mit höherem Widerstand umfasst. Ferner ist die äussere Hülse 5 an ihrem in den Wirbelkörper 12 eingeführten Ende geschlossen. Aufgrund des höheren Widerstands erwärmt und verformt sich während des Stromdurchflusses durch den Pin 2 die äussere Hülse 5.

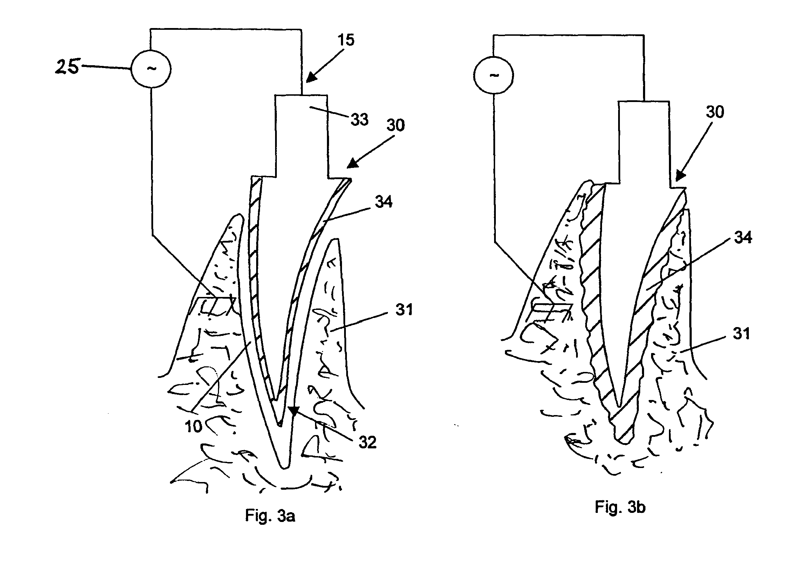

Die in den Fig. 3a und 3b dargestellte Ausführungsform umfasst ein Zahnimplantat 30 aus Titan, dessen in den Knochen 31 einzuführender Abschnitt mit einer Schicht 34 aus einem leitfähigem Thermoplast umgeben ist. Hierzu wird der in den Kochen 31 einzuführende Abschnitt des Zahnimplantates 30 mehrfach eine Lösung aus Poly-D,L-lactid mit 25% Carbon Black getaucht und zwischen den Tauchgängen getrocknet. Das vom distalen Ende 32 weggewandte, nicht beschichtete Ende 33 wird an eine Stromquelle 25 angeschlossen. Das Zahnimplantat 30 wird in das mit Untermass vorgebohrte Loch 10 gesetzt und der Strom eingeschaltet (Fig. 3a). Sobald die Stromübertragung durch die Elektrode 15, das Zahnimplantat 30, die Schicht 34 aus einem Polymer und den Knochen 31 erfolgt, erweicht die Schicht 34 vom distalen Ende 32 her und das Zahnimplantat 30 kann nun mittels Druck in die Tiefe des Lochs 10 gedrückt werden. Beim Einpressen des Zahnimplantates 30 in das Loch 10 wird der die Schicht 34 bildende Thermoplast in die Zwischenräume im Knochen 31 gepresst, so dass eine mechanische Verbindung zwischen dem Zahnimplantat 30 und dem Knochen 31 geschaffen wird. Das Erstarren des Polymers, d.h. der Schicht 34 im Knochen 31 führt zu einer primären, belastungsstabilen Verbindung zwischen Knochen 31 und Zahnimplantat 30 (Fig. 3b).

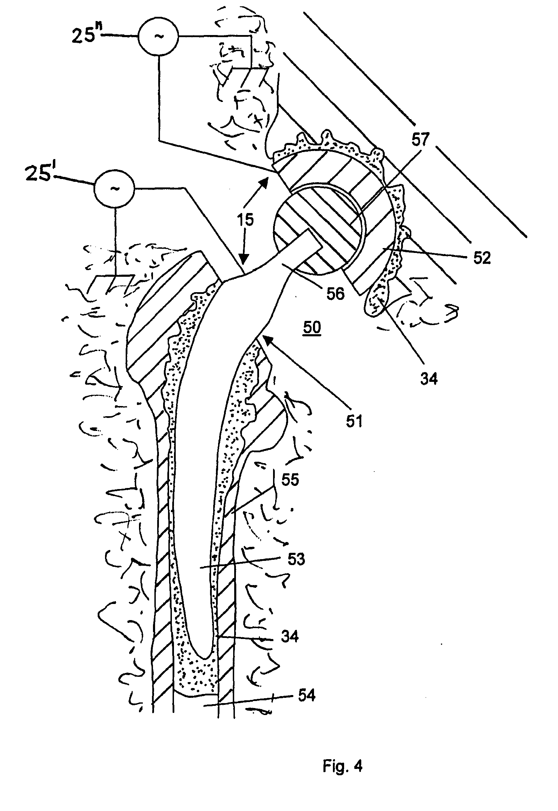

Die in Fig. 4 dargestellte Ausführungsform umfasst ein als Hüftgelenkprothese 50 ausgebildetes medizinisches Implantat. Die Hüftgelenkprothese 50 umfasst eine elektrisch leitende metallische Femurkomponente 51, deren in den Markraum 54 des Femurs 55 einzuführender Schaft 53 analog zu der in Fig. 3a und 3b dargestellten Ausführungsform mit einer Schicht 34 aus einem leitfähigen Polymer beschichtet ist, und eine ebenfalls leitende metallische Gelenkschale 52, welche an ihrer in die Gelenkpfanne berührenden Aussenfläche mit einer Schicht 34 aus einem leitfähigen Polymer beschichtet ist. Die Femurkomponente 51 wird an ihrem nicht beschichteten Hals 56 oder Gelenkkopf 57 an eine Stromquelle 25' angeschlossen. Analog wird die Gelenkschale 52 an eine zweite Stromquelle 25" angeschlossen. Die Femurkomponente 51 wird in den mit Untermass aufgebohrten Markraum 54 eingeführt und die Gelenkschale 52 in die Gelenkpfanne eingesetzt. Sobald der Strom eingeschaltet ist und die Stromübertragung über die Elektrode 15, die Femurkomponente 51, die Schicht 34 aus einem Polymer und den Femur 55 erfolgt, erweicht die Schicht 34 durch inner Wärmeentwicklung. Im zweiten Stromkreis erfolgt die Stromübertragung über eine zweite Elektrode 15, die Gelenkschale 52 und den Beckenknochen, wodurch die Schicht 34 aussen an der Gelenkschale 52 durch inner Wärmeentwicklung erweicht. Die Femurkomponente 51 kann nun mittels Druck in die Tiefe des Markraums 54 gedrückt werden. Beim Einpressen der Femurkomponente 51 in den Markraum 54 wird der die Schicht 34 bildende Thermoplast in die Zwischenräume im Knochen gepresst, so dass eine mechanische Verbindung zwischen der Femurkomponente 51 und dem Knochen geschaffen wird. Analog dazu wird die Gelenkschale 52 in die Gelenkpfanne gedrückt, wobei die erweichte Schicht 34 an der Gelenkschale 52 in die Zwischenräume im Knochen gepresst wird und ebenfalls eine mechanische Verbindung zwischen der Gelenkschale 52 und dem Knochen geschaffen wird. Das Erstarren des Polymers, d.h. der Schichten 34 an der Femurkomponente 51 im Femur 55 und an der Gelenkschale 52 in der Gelekpfanne führt zu einer primären, belastungsstabilen Verbindung zwischen Knochen und Hüftgelenkprothese 50.

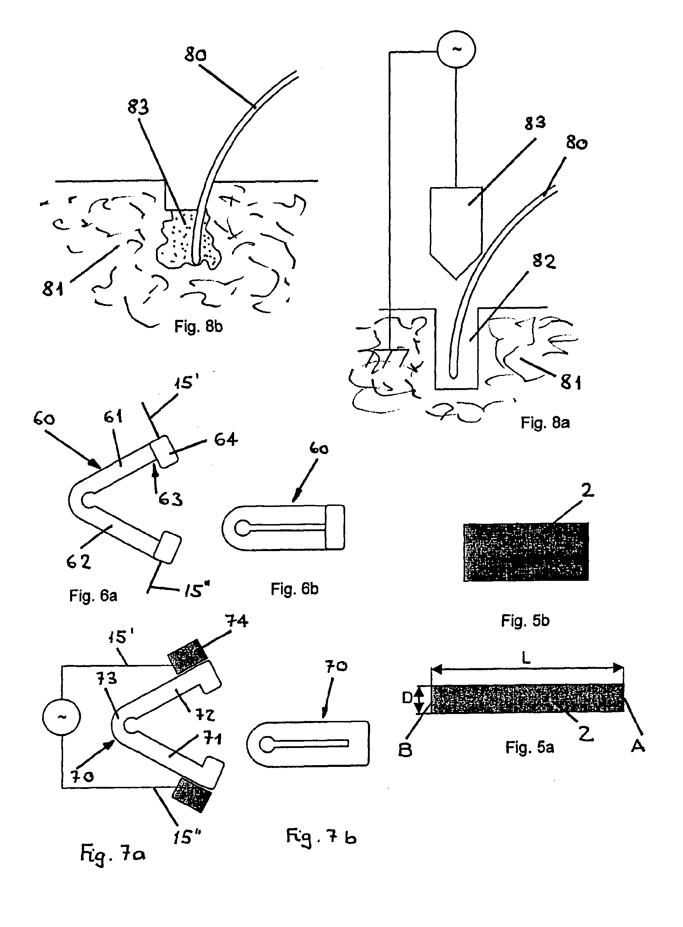

In den Fig. 5a und 5b ist eine weitere Ausführungsform dargestellt, wobei der Pin 2 durch ein geeignetes Herstellungsverfahren, z.B. mittels Spritzgiessen, mit einer Eigenspannung versehen wird und im erkalteten Zustand eine Länge L und einen Durchmesser D aufweist (Fig. 5a). Durch das Erwärmen des gesamten Pins 2 mittels Stromdurchfluss zwischen den Polen A,B relaxiert der Thermoplast und der Pin 2 wird kürzer und nimmt im Durchmesser zu (Fig. 5b), was zu einer Fixation im oder am umgebenden Gewebe führt.

In der in den Fig. 6a und 6b dargestellten Ausführungsform ist das medizinische Implantat als Clip 60 ausgebildet. Der Clip 60 ist U-förmig ausgebildet und umfasst zwei Arme 61,62 deren freie Enden 63 je ein aus einem leitfähigen Polymer bestehendes Element 64 umfassen. Diese gegenüber den Armen 61,62 dickeren Elemente 64 werden mittels Elektroden 15',15" an einen Stromkreis angeschlossen (Fig. 6a). Nach dem Einschalten der Stromquelle wird der Clip 60 zusammengepresst, d.h. die beiden Elemente 64 werden aneinander gepresst. Durch den Stromfluss werden die beiden Elemente 64 erwärmt und erweichen an ihren aneinander anliegenden Kontaktstellen und können so durch ein Aufbringen von Druck zusammengefügt und durch Verschmelzen miteinander verbunden werden (Fig. 6b).

Der in den Fig. 7a und 7b dargestellte Clip 70 unterscheidet sich von dem in den Fig. 6a und 6b dargestellten Clip nur darin, dass der Clip 70 einstückig aus einem leitfähigen Polymer hergestellt ist. Die Arme 71,72 werden mit einer bipolaren Klemme 74 gegriffen, über je eine Elektrode 15',15" unter Strom gesetzt und zusammengedrückt. Durch den Stromfluss erweicht das die Arme 71,72 verbindende Scharnier 73 und erlaubt ein Verbiegen des Clips 70. Beim Aufeinandertreffen der dem Scharnier 73 abgewandten Enden der Arme 71,72 wird auch hier Strom übertragen was zu einem Aufschmelzen und gewünschten Verbinden der beiden Arme 71,72 an den gegenüber den Armen 71,72 verdickten Enden führt.

In der in den Fig. 8a und 8b dargestellten Ausführungsform umfasst das medizinische Implantat einen Faden 80, welcher aus einem Material mit hohem Schmelzpunkt besteht, und einen Anker 83 aus einem leitfähigen Polymer. Der Faden 80 soll am Knochen 81 fixiert werden, um mit dem Faden 80 z.B. eine Sehne oder ein anderes Knochenstück zu halten. Dafür wird ein Loch 82 mit einem Durchmesser von 3mm 15mm tief in den Knochen 81 gebohrt. In dieses Loch 82 im Knochen 81 wird der Faden 80 eingeführt. Anschliessend wird auf das Loch 82 ein Anker 83 aufgesetzt, welcher im Durchmesser etwas dicker ist als das Loch 82. Analog zum Beispiel 1 wird auch hier der Anker 83 mittels Elektrokauter unter Strom gesetzt und nach Aufweichen durch den Stromfluss in den Knochen 81 gedrückt. Nach Ausschalten des Stroms, erstarrt das leitfähige Polymer und der Anker 83 ist zusammen mit dem Faden 80 im Knochen 81 fixiert.

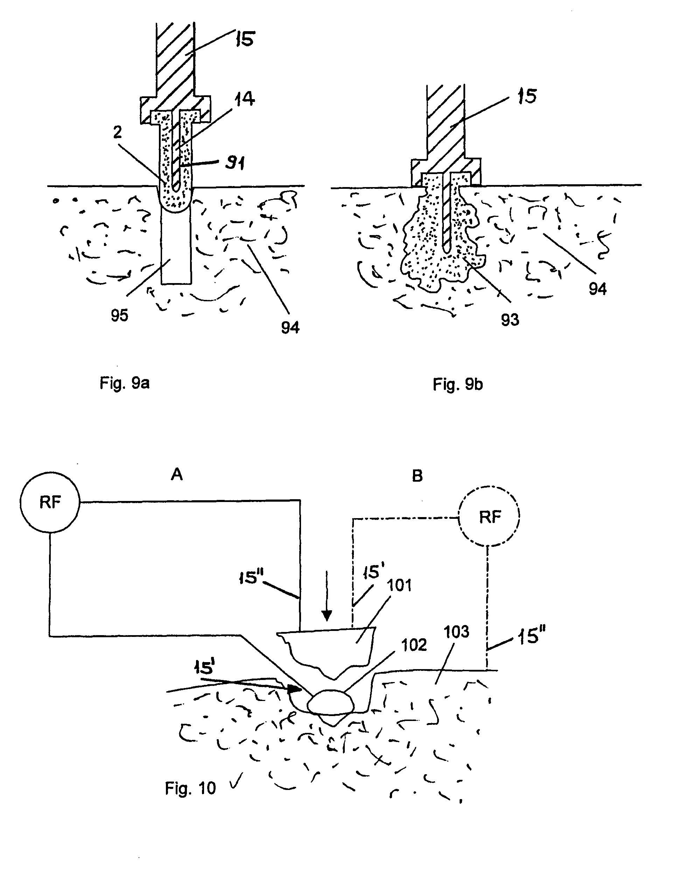

In den Fig. 9a, 9b ist eine Ausführungsform des medizinischen Implantates dargestellt, welche zum Füllen von Defekten an Knochen 94 geeignet ist. Analog zur Ausführungsform gemäss Fig. 1 wird ein Pin 2 verwendet, welcher einen zentralen, an der Spitze des Pins 2 geschlossenen Hohlraum 91 zur Aufnahme eines mit der Elektrode 15 verbundenen Metalistiftes 14 aufweist. Der Metallstift 14 kann nach dem Verschmelzen des Pins 2 wieder entfernt werden oder auch aus einem resorbierbaren Material gefertigt sein. Zum Füllen z.B. eines Tibiakopfdefekts wird bei einem Patienten mit Tibiakopffraktur ein 4mm Loch 95 von ventral durch die Kortikalis zum Defekt hin gebohrt (Länge 2cm). Durch dieses Loch 95 wird darauf der Pin 2 zusammen mit dem Metallstift 14 in die Markhöhle und den spongiösen Raum des Knochens unter Stromanwendung geschoben, und so wie bei einer Verbundosteosynthese durch Verschmelzen des Pins 2 zur einer Füllung 93 ein stabiler Knochen geschaffen. Die danach in diese Füllung 93 eingebrachten Schrauben (nicht gezeichnet) halten im zuvor geschmolzenen und nachher erstarrten Polymer vorzüglich.

In Fig. 10 ist eine Ausführungsform dargestellt, in welcher das Polymer des medizinischen Implantates als Perle 102 ausgebildet ist. Diese Perle 102 kann in den Hohlraum eingefügt werden, welcher beim Herausbrechen eines Knochenfragmentes 101 aus einem Knochen 103 entsteht. Das Einpassen des Knochenfragments 101 in den Hohlraum und Verbinden des Knochenfragments 101 mit dem Knochen 103 mittels Schmelzen der Perle 102 und Pressen des Polymers in die Zwischenräume im Knochenfragment 101 und im Knochen 103 kann durch zwei verschiedene Varianten A und B erreicht werden. Bei der Variante A wird eine erste Elektrode 15' mit der Perle 102 verbunden während eine zweite Elektrode 15" am Knochenfragment 101 befestigt wird. Nach Einschalten des Stromquelle erfolgt die Stromübertragung von der Stromquelle über die erste Elektrode 15', die Perle 102, unter Erwärmung derselben durch diese hindurch und über die zweite Elektrode 15". Bei der Variante B wird die erste Elektrode 15' am Knochenfragment 101 befestigt während die zweite Elektrode 15" am Knochen 103 befestigt wird. Hier erfolgt die Stromübertragung nach Einschalten der Stromquelle über die erste Elektrode 15', das Knochenfragment 101, die Perle 102 unter Erwärmung derselben, den Knochen 103 und die zweite Elektrode 15".

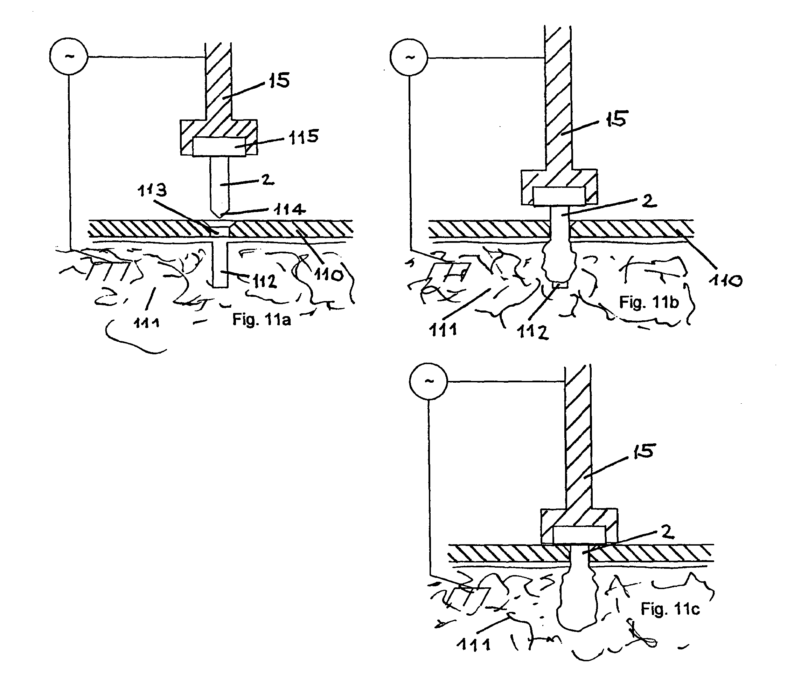

Die in den Fig. 11a - 11c dargestellte Ausführungsform umfasst einen aus einem leitfähigen Polymer hergestellten Pin 2, welcher zur Fixation einer Knochenplatte 110 an einem Knochen 111 geeignet ist. Die Knochenplatte 110 ist eine resorbierbare Osteosyntheseplatte mit einer Dicke von 1 mm aus einem Poly-D,L-Lactid. Zur Fixation einer Fraktur wird die Knochenplatte 110 auf die zu fixierenden Knochenfragmente aufgebracht und die für deren Fixation am Knochen 111 benötigten Löcher 112 in den Knochen 111 gebohrt. In diesem Beispiel handelt es sich um eine Knochenplatte 110 mit Schraubenlöchern 113 für 2.0 mm Schrauben. Die in den Knochen 111 gebohrten Löcher 112 haben einen Durchmesser von 1.5 mm. Der elektrisch leitende Pin 2 hat einen Durchmesser von 2.0 mm wird mit seinem hinteren, im Durchmesser vergrösserten Kopf 115 an eine Elektrode 15 angebracht, welche an einen handelsüblichen Elektrokauter angeschlossen ist. Die neutrale Elektrode des Elektrokauters wird am Körper des Patienten auf konventionelle Weise angeschlossen (Fig. 11a). Der Pin 2 wird mit seiner in den Knochen 111 einzubringenden Spitze 114 durch das Schraubenloch 113 in der Knochenplatte 110 geführt und auf das im Knochen 11 vorgebohrte Loch 112 aufgesetzt und unter Strom gesetzt. Die Stromübertragung durch den elektrisch leitenden Pin 2 erwärmt diesen. Da der grösste elektrische Spannungsabfall am Übergang zwischen Knochen 111 und Pin 2 liegt, entsteht hier im Pin 2 die grösste Wärme, wodurch der Pin 2 vor allem an seiner Oberfläche aufgeweicht wird. Durch Druck auf die Elektrode 15 wird der Pin 2 in das in den Knochen 111 vorgebohrte Loch 112 geschoben und das thermoplastische Material fliesst in die zugänglichen intertrabekulären Zwischenräume im spongiösen Knochen (Fig. 11b). Nach Ausschalten des Stroms kühlt das Polymer wieder ab und erstarrt. Der im Durchmesser gegenüber dem Schraubenloch 113 in der Knochenplatte 110 grössere Kopf 115 des Pins 2 hält nun die Knochenplatte 110 (Fig. 11c).

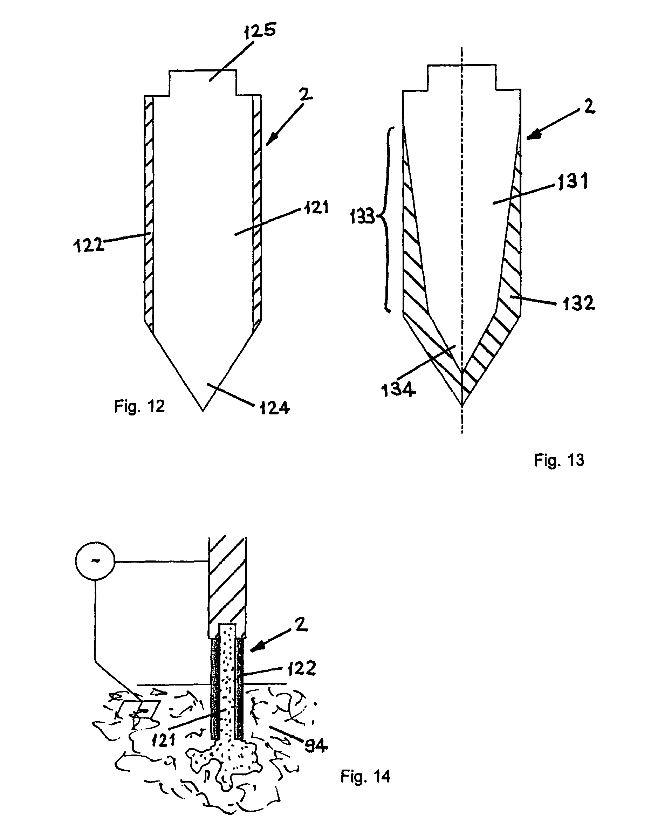

Fig. 12 und 13 zeigen je einen Pin 2, welcher einen Kern 121,131 aus einem Material mit einem tiefen Widerstand, beispielsweise aus einem Metall oder einem leitenden Polymer und einer Beschichtung 122,132 aus einem elektrisch leitenden Polymer mit höherem Widerstand besteht. Die Beschichtung 122 in Fig. 12 ist hülsenförmig ausgebildet und erstreckt sich auf dem zylindrischen Teil 123 des Pins 2. Die Spitze 124 des Pins 2 sowie das axial entgegengesetzte hintere Ende 125, welches an eine Elektrode anschliessbar ist, sind ohne Beschichtung 122 ausgebildet. Die Beschichtung 132 in Fig. 13 ist nur partiell an einem vorderen Abschnitt 133 des Pins 2 angebracht, und umschliesst den sich verjüngenden Abschnitt 133 des Pins 2 inklusive dessen Spitze 134. Ein gemäss den Fig. 12 oder 13 ausgebildeter Pin 2 ermöglicht eine selektive Erwärmung eines Thermoplasten um eine Verformung zu erreichen. Der Pin 2 wird sich in Fig. 12 an der dünnen Spitze 124 erwärmen, da die Hülse als Isolator wirkt und der Strom über die Spitze abfliesst. In Fig. 13 wird sich Pin 2 an der Zone mit grösserem Widerstand im Stromkreis, d.h. an der Beschichtung 132 erwärmen und verformen.

Fig. 14 zeigt die Anwendung eines Pins 2 gemäss Fig. 12 bei der in den Fig. 9a und 9b beschriebenen Füllung eines Defektes an einem Knochen 94.

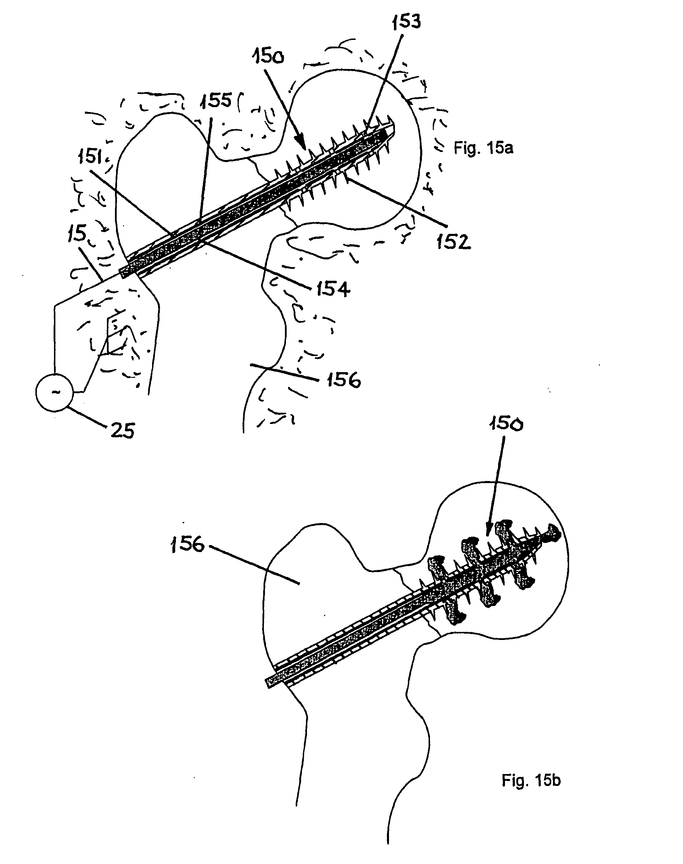

Fig. 15a und 15b zeigen eine Ausführungsform, worin das medizinische Implantat eine dynamische Hüftschraube 150 und einen aus einem leitfähigen Polymer bestehenden Pin 2 umfasst. Die dynamische Hüftschraube 150 hat einen hohlen Schaft 151 mit einem Knochengewinde 152 am vorderen, bis in den Hüftgelenkkopf reichenden Ende. Im Bereich des Knochengewindes 152 sind radiale Perforationen 153 angeordnet, welche den Schaft 151 zwischen dessen zentralem Hohlraum 154 und seiner Peripherie radial durchdringen. Der Hohlraum 154 ist ausser im Bereich der Perforationen 153 mit einer isolierenden Beschichtung 155 versehen. Die dynamische Hüftschraube 150 wird im Rahmen einer Schenkelhalsfraktur bei Osteoporose durch den Schenkelhals implantiert. In den zentralen Hohlraum 154 wird ein, wie unter Beispiel 9 beschrieben, isolierter Pin 2 von 2.9 mm Durchmesser eingeführt und an seinem hinteren, dem Knochengewinde 152 der dynamischen Hüftschraube 150 entgegengesetzten Ende mittels einer Elektrode 15 an eine Stromquelle 25 angeschlossen. Unter Stromanwendung schmilzt so der Pin 2 im Innern der Hüftschraube 150 und das verflüssigte Polymer dringt durch die Perforationen 153 nach aussen in den Knochen 156 und schafft so eine Augmentation des Knochens 156 in welchem das Implantat hält. Nach Aushärten des Polymers ist die Hüftschraube 150 belastungsstabil (Fig. 15b).

## Patentansprüche

1. Medizinisches Implantat, welches mindestens teilweise ein körperverträgliches, elektrisch leitendes Polymer mit dem elektrischen spezifischen Widerstand p umfasst, mit der Eigenschaft, dass es durch einen Stromfluss durch das Polymer erwärmbar und erweichbar ist,
**dadurch gekennzeichnet, dass**
a) der elektrische spezifische Widerstand p grösser als 500 Ohm·cm ist;
b) das Polymer eine Volumenleitfähigkeit von mindestens 10⁻⁴ S/m aufweist; und
c) das Polymer einen Oberflächenwiderstand von mindestens 10⁻¹ Ohm/square, aufweist.

2. Medizinisches Implantat nach Anspruch 1, **dadurch gekennzeichnet, dass** das Polymer derart ausgewählt ist, dass die Erweichung unterhalb einer Erwärmungstemperatur von 250°C stattfindet.

3. Medizinisches Implantat nach Anspruch 1 oder 2, **dadurch gekennzeichnet, dass** ausser dem Polymer selbst keine anderen Bauteile des Implantats für die Erwärmung des Implantats vorgesehen sind.

4. Medizinisches Implantat nach einem der Ansprüche 1 bis 3, **dadurch gekennzeichnet, dass** es Mittel zur Befestigung einer Elektrode umfasst.

5. Medizinisches Implantat nach Anspruch 1, **dadurch gekennzeichnet, dass** der elektrische spezifische Widerstand p grösser als 1500 Ohm·cm ist.

6. Medizinisches Implantat nach einem der Ansprüche 1 bis 5, **dadurch gekennzeichnet, dass** das Polymer einen Oberflächenwiderstand von mindestens 10² Ohm/square aufweist.

7. Medizinisches Implantat nach einem der Ansprüche 1 bis 6, **dadurch gekennzeichnet, dass** es neben dem Polymer noch Implantatteile aus weiteren Materialien umfasst, vorzugsweise aus folgenden Gruppen ausgewählt: Metalle, Carbon, Keramik, PEEK, nicht thermoplastische Polymere, welche vorzugsweise aus der Gruppe der Polymethylmethacrylate ausgewählt werden und/oder anorganische Materialien, so wie Kalziumphosphat, Kalziumsulfat oder Knochenzement.

8. Medizinisches Implantat nach einem der Ansprüche 1 bis 7, **dadurch gekennzeichnet, dass** die elektrisch Leitfähigkeit des Polymers durch geeignete Dotierung erreicht wird.

9. Medizinisches Implantat nach einem der Ansprüche 1 bis 8, **dadurch gekennzeichnet, dass** das Polymer mit einer elektrisch leitfähigen Keramik kombiniert ist.

10. Medizinisches Implantat nach Anspruch 9, **dadurch gekennzeichnet, dass** die Keramik eine glasartige oder amorphe Struktur aufweist.

11. Medizinisches Implantat nach einem der Ansprüche 1 bis 10, **dadurch gekennzeichnet, dass** das Polymer durch elektrisch leitende Zusatzstoffe zu einem elektrisch nichtleitenden Polymer erhalten wird.

12. Medizinisches Implantat nach Anspruch 11, **dadurch gekennzeichnet, dass** die elektrisch leitenden Zusatzstoffe aus jedem möglichen elektrisch leitenden festen oder flüssigen Material in Form von Partikeln, Körnchen, Partikelanhäufungen irgendeiner äusseren Form bestehen.

13. Medizinisches Implantat nach Anspruch 11 oder 12, **dadurch gekennzeichnet, dass** die elektrisch leitenden Zusatzstoffe aus folgenden Materialien gewählt werden:
- Metallische Materialien z.B. Eisen, Magnesium, Gold, Silber, Legierungen oder Amalgame;
- Carbonpartikel z.B. Ruß, Carbon Nanoröhren (carbon nanotubes), Fullerene;
- Salze oder Substanzen, die Wasser in einer schnellen Weise anziehen können, um den Fluss von Elektrizität in der erforderlichen Menge zuzulassen. Solche Salze können aus z.B. Natriumchlorid, Natrium Sulfat oder Magnesiumsalzen bestehen.
- Elektrisch leitende Polymere z.B. Polymere der Pyrrole, Aniline, Dialkylfluoride, Thiophene oder Ethylendioxythiophene gewählt werden.
- biokompatible Öle, vorzugsweise Silikone;
- wässerige Lösungen, vorzugsweise Salzlösungen.

14. Medizinisches Implantat nach einem der Ansprüche 1 bis 13, **dadurch gekennzeichnet, dass** das gesamte Implantat oder nur das Polymer teilweise mit nicht elektrisch leitenden Materialien beschichtet ist.

## Claims

1. A medical implant, comprising at least partially a body-compatible, electrically conductive polymer having the electrical resistivity p, with the property that it can be warmed and softened by flowing a current through said polymer,
**characterized in that**
a) the specific electrical resistivity p is greater than 500 ohm • cm;
b) the polymer has a volume conductivity of at least 10⁻⁴ S/m; and
c) the polymer has a surface resistivity of at least 10⁻¹ ohm/square.

2. The medical implant according to claim 1, **characterized in that** the polymer is selected such that the softening takes place below a warming temperature of 250°C.

3. The medical implant according to claim 1 or 2, **characterized in that** no other components of the implant are provided for the warming of the implant in addition to the polymer itself.

4. The medical implant according to any one of claims 1 to 3, **characterized in that** it comprises means for mounting an electrode.

5. The medical implant according to claim 1, **characterized in that** the specific electrical resistivity p is greater than 1500 ohm • cm.

6. The medical implant according to any one of claims 1 to 5, **characterized in that** the polymer has a surface resistivity of at least 10² ohm/square.

7. The medical implant according to any one of claims 1 to 6, **characterized in that** it also comprises implant components made of other materials in addition to the polymer, preferably selected from the following groups: metals, carbon, ceramic, PEEK, non-thermoplastic polymers preferably selected from the group of polymethyl methacrylates and/or inorganic materials such as calcium phosphate, calcium sulfate or bone cement.

8. The medical implant according to any one of claims 1 to 7, **characterized in that** the electrically conductivity of the polymer is achieved by appropriate doping.

9. The medical implant according to any one of claims 1 to 8, **characterized in that** the polymer is combined with an electrically conductive ceramic.

10. The medical implant according to claim 9, **characterized in that** the ceramic has a glassy or amorphous structure.

11. The medical implant according to any one of claims 1 to 10, **characterized in that** the polymer is obtained by electrically conductive additives to a non-conductive polymer.

12. The medical implant according to claim 11, **characterized in that** the electrically conductive additives are composed of any possible electrically conductive solid or liquid material in the form of particles, granules, particle accumulations of any outer shape.

13. The medical implant according to claim 11 or 12, **characterized in that** the electrically conductive additives are selected from the following materials:
- metallic materials, e.g., iron, magnesium, gold, silver, alloys or amalgams;
- carbon particles, e.g., carbon black, carbon nanotubes (carbon nanotubes), fullerenes;
- salts or substances that can attract water rapidly, to permit the flow of electricity in the amount necessary. Such salts can be, e.g., sodium chloride, sodium sulfate or magnesium salts. -electrically conductive polymers, e.g., polymers of pyrroles, anilines, dialkylfluorides, thiophenes or ethylenedioxythiophenes [selected]. - biocompatible oils, preferably silicones; - aqueous solutions, preferably salt solutions.

14. The medical implant according to any one of claims 1 to 13, **characterized in that** the entire implant or only the polymer partially is coated with electrically non-conductive materials.

## Revendications

1. Implant médical, qui comprend, au moins en partie, un polymère compatible avec l'organisme, électriquement conductible, avec la résistance électrique spécifique p, et qui a la propriété de pouvoir être réchauffé et ramolli par un flux de courant électrique traversant le polymère,
**caractérisé en ce que**
a) la résistance électrique spécifique p est supérieure à 500 ohm•cm;
b) le polymère présente une conductivité volumique d'au moins 10⁻⁴ S/m; et
c) le polymère présente une résistance surfacique d'au moins 10⁻¹ ohm/carré.

2. Implant médical selon la revendication 1, **caractérisé en ce que** le polymère est sélectionné de telle sorte que le ramollissement a lieu au-dessous d'une température de chauffage de 250°C.

3. Implant médical selon la revendication 1 ou 2, **caractérisé en ce qu'**en dehors du polymère lui-même, aucun autre composant de l'implant n'est prévu pour le chauffage de l'implant.

4. Implant médical selon l'une des revendications 1 à 3, **caractérisé en ce qu'**il comprend un moyen destiné à la fixation d'une électrode.

5. Implant médical selon la revendication 1, **caractérisé en ce que** la résistance électrique spécifique p est supérieure à 1500 ohm•cm.

6. Implant médical selon l'une des revendications 1 à 5, **caractérisé en ce que** le polymère présente une résistance surfacique d'au moins 10² ohm/carré.

7. Implant médical selon l'une des revendications 1 à 6, **caractérisé en ce qu'**il comprend aussi, en plus du polymère, des parties d'implant composées d'autres matériaux, sélectionnés de préférence parmi les groupes suivants : métaux, carbone, céramique, PEEK, polymères non thermoplastiques, lesquels sont sélectionnés de préférence parmi le groupe des polyméthacrylates de méthyle et/ou de matériaux anorganiques, ainsi que de phosphate de calcium, de sulfate de calcium ou de ciment osseux.

8. Implant médical selon l'une des revendications 1 à 7, **caractérisé en ce que** la conductibilité électrique du polymère est atteinte par un dosage adéquat.

9. Implant médical selon l'une des revendications 1 à 8, **caractérisé en ce que** le polymère est combiné avec une céramique électriquement conductible.

10. Implant médical selon la revendication 9, **caractérisé en ce que** la céramique présente une structure vitreuse ou amorphe.

11. Implant médical selon l'une des revendications 1 à 10, **caractérisé en ce que** le polymère est obtenu par des additifs électriquement conductibles ajoutés à un polymère électriquement non conductible.

12. Implant médical selon la revendication 11, **caractérisé en ce que** les additifs électriquement conductibles sont composés de chaque matériau solide ou liquide possible, étant électriquement conductible, sous forme de particules, de granulés, d'agglomérations de particules se présentant sous quelque forme externe que ce soit.

13. Implant médical selon la revendication 11 ou 12, **caractérisé en ce que** les additifs électriquement conductibles sont sélectionnés parmi les matériaux suivants :
- matériaux métalliques, par exemple fer, magnésium, or, argent, alliages ou amalgames ;
- particules de carbone, par exemple suie, nanotubes de carbone (carbon nanotubes), fullerènes ;
- sels ou substances ayant un pouvoir d'attraction rapide de l'eau afin de permettre la circulation du courant électrique dans la quantité nécessaire de tels sels pouvant être composés par exemple de chlorure de sodium, de sulfate de sodium ou de sels de magnésium.
- polymères électriquement conductibles, par exemple polymères sélectionnés parmi les groupes du pyrrole, de l'aniline, du dialkyle fluorure, du thiophène ou de l'éthylène dioxythiophène.
- huiles biocompatibles, de préférence des silicones ;
- solutions aqueuses, de préférence des solutions salines.

14. Implant médical selon l'une des revendications 1 à 13, **caractérisé en ce que** la totalité de l'implant, ou seulement le polymère en partie, est revêtu de matériaux qui ne sont pas électriquement conductibles.
